(19) Europäisches Patentamt
European Patent Office
Office européen des brevets

(11) EP 3 715 411 A1

(12) EUROPEAN PATENT APPLICATION
published in accordance with Art. 153(4) EPC

(43) Date of publication:
**30.09.2020 Bulletin 2020/40**

(21) Application number: **18880526.1**

(22) Date of filing: **20.11.2018**

(51) Int Cl.:
***C08L 27/06*** *(2006.01)* ***C08K 5/12*** *(2006.01)*

(86) International application number:
**PCT/JP2018/042838**

(87) International publication number:
**WO 2019/102995 (31.05.2019 Gazette 2019/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.11.2017 JP 2017224286**
**05.12.2017 JP 2017233429**
**19.09.2018 JP 2018174948**

(71) Applicant: **New Japan Chemical Co., Ltd.**
**Kyoto 612-8224 (JP)**

(72) Inventors:
- **YOSHICHIKA, Shoki**
  **Kyoto-shi**
  **Kyoto 612-8224 (JP)**
- **MIYAZAKI, Ken-ichi**
  **Kyoto-shi**
  **Kyoto 612-8224 (JP)**
- **TSUJI, Taiki**
  **Kyoto-shi**
  **Kyoto 612-8224 (JP)**

(74) Representative: **Herzog IP Patentanwalts GmbH**
**Immermannstraße 40**
**40210 Düsseldorf (DE)**

# (54) METHOD TO IMPROVE VISIBILITY OF CONTENTS, VINYL CHLORIDE-BASED RESIN COMPOSITION, STABILIZER, VINYL CHLORIDE-BASED RESIN MOLDED BODY, MEDICAL MATERIAL, AND STERILIZATION PROCESSING METHOD

(57) A purpose of the present invention is to provide a method to improve the visibility of the contents of a material containing a vinyl chloride-based resin molded body that has been subjected to electron beam irradiation sterilization, as well as to provide a medical material that has improved visibility of the contents thereof after electron beam irradiation sterilization. By including a stabilizer that contains 4-cyclohexene-1,2-dicarboxylic acid diester and/or 4,5-epoxycyclohexane-1,2-dicarboxylic acid diester, loss of transparency and staining that occur over time during and after electron beam irradiation sterilization can be reduced, and a medical material that contains a vinyl chloride-based resin molded body and has improved visibility of the contents thereof after electron beam irradiation sterilization can be obtained.

EP 3 715 411 A1

## Description

TECHNICAL FIELD

The present invention relates to a method for improving visibility of contents of a material containing a vinyl chloride-based resin molded body that has been subjected to electron beam irradiation sterilization. Specifically, the present invention relates to the above method, in which the vinyl chloride-based resin molded body includes a stabilizer containing a 4-cyclohexene-1,2-dicarboxylic acid diester and/or a 4,5-epoxycyclohexane-1,2-dicarboxylic acid diester for improving the visibility of contents of a medical material after electron beam irradiation sterilization. The present invention further relates to a medical material having an improved visibility of contents after electron beam irradiation sterilization with the stabilizer. In addition, the present invention relates to a vinyl chloride-based resin molded body for a medical material sterilized by radiation irradiation, and to a sterilization processing method by radiation irradiation of the vinyl chloride-based resin molded body. More specifically, the present invention relates to a vinyl chloride-based resin molded body for a medical material, in which coloring of the medical material after sterilization processing by radiation irradiation, particularly electron beam irradiation, can be suppressed, and the medical material can maintain a high transparency even after sterilization processing by electron beam irradiation, and to a sterilization processing method by radiation irradiation of the vinyl chloride-based resin molded body.

BACKGROUND ART

Vinyl chloride-based resin has good processability, and is excellent in transparency and safety, and furthermore excellent in chemical resistance and durability, and can be adjusted to various flexibility and hardness levels by blending a plasticizer and therefore is used in a wide variety of applications. In particular, a soft vinyl chloride-based resin composition containing a plasticizer is superior in kink resistance compared to polyolefins and the like, and is widely used as a medical material such as a medical tube such as a catheter or a medical bag such as a blood bag and an infusion bag. Phthalic acid esters such as di-2-ethylhexyl phthalate (hereinafter sometimes abbreviated as DEHP or DOP) are used as a plasticizer in the medical material (Patent Document 1) .

Medical materials, such as medical tubes and blood bags, need to be harmless to the human body, to maintain sterility until use, and to be able to check the content situation. Therefore, the medical material is generally used after being sterilized by various methods before use from the viewpoint of hygiene. Examples of the sterilization method include dry heating, boiling, heat sterilization such as pressurized hot water treatment, electron beam irradiation sterilization such as irradiation with γ-rays and electron beams, and chemical sterilization such as chemical treatment with ethylene oxide gas. In the case of chemical sterilization, there is a concern that toxic ethylene oxide gas may remain, and in the case of heat sterilization, there is a concern that problems such as deformation of medical materials at high temperatures may occur. Thus, radiation irradiation sterilization such as electron beam irradiation sterilization has been used as the safest method.

Radiation irradiation sterilization includes γ-ray irradiation sterilization using cobalt 60 as a radiation source and electron beam irradiation sterilization using an electron accelerator. In the past, γ-ray irradiation sterilization, which is superior in terms of equipment, was the mainstream, but electron beam irradiation sterilization has become the mainstream in recent years from the viewpoint of concerns about the supply of cobalt 60 as a radiation source and environmental problems such as disposal of waste.

Electron beam irradiation sterilization has a very high dose rate (amount of radiation irradiated per unit time) compared to conventional γ-ray irradiation sterilization, and another major feature is that sterilization can be completed in an extremely short time, i.e. one-tenth of the time required for γ-ray irradiation sterilization.

On the other hand, radiation irradiation sterilization may cause degradation of resin due to its strong sterilizing power. The same applies to the above-mentioned vinyl chloride-based resin, in which a decomposition reaction or the like due to a dehydrochlorination reaction is apt to occur, and as a result, there is a problem that, after sterilization, remarkable coloring occurs with time and transparency is lowered. A significant coloring and a decrease in transparency make it difficult to identify the contents, which may cause medical errors. In recent years, medical accidents frequently occur in hospitals and the like, and such a remarkable reduction in coloring and transparency has become a fatal defect as a medical material. At present, such a problem is more serious particularly in electron beam irradiation sterilization with a high dose rate than in conventional γ-ray irradiation sterilization.

Various methods have been proposed and are practically used to suppress the degradation of the vinyl chloride-based resin due to the decomposition described above. For example, organic compounds of heavy metals such as lead and cadmium are known to provide a large stabilizing effect. However, since these heavy metal organic compounds are highly toxic and harmful to the human body, they cannot be used for medical materials.
Therefore, metal soap-based stabilizers such as calcium soap, zinc soap, etc. and organic tin-based stabilizers, which are said to have relatively low toxicity as metal-based organic compounds at present, are often used in medical materials.

However, increasing the amount of the metal-based stabilizer decreases the transparency and the like, and it is therefore difficult to increase the amount of the metal-based stabilizer, so that the effect of the metal-based stabilizer cannot be said to be sufficient for electron beam irradiation sterilization during which severe degradation occurs (Patent Document 2).

Further, epoxidized vegetable oil-based compounds such as epoxidized soybean oil and epoxidized linseed oil are also said to be effective in suppressing coloring due to the degradation described above, and are used in combination with metal-based stabilizers such as zinc soap and calcium soap (Patent Document 3). Particularly, a vinyl chloride-based resin composition in which DEHP and an epoxy compound are combined is disclosed. However, similarly to the above metal-based stabilizers, in radiation irradiation sterilization, especially in electron beam irradiation sterilization with a high dose rate, it is necessary to increase the blending amount in order to suppress the degradation, which causes a new problem such as bleeding. Therefore, it is difficult to obtain a satisfactory effect (Patent Document 4).

In addition, DEHP, which is a plasticizer conventionally used, has a problem not only in its coloring and reduction of transparency, but also in its elution when a medical device is used. Therefore, applications of DEHP alternative plasticizers such as trimellitic acid triesters represented by tri-2-ethylhexyl trimellitate (TOTM) and cyclohexanedicarboxylic acid diesters represented by diisononyl 1,2-cyclohexanedicarboxylate (DINCH) to medical devices have been studied, but there is a very large problem of coloring due to sterilization processing (Patent Document 2).

Also, with respect to this coloring problem, there is a disclosure that coloring is suppressed by blending a silane compound as a radiation-resistant agent with a tetrahydrophthalic acid diester-based plasticizer. However, particularly in the case of a high-dose electron beam, a sufficiently satisfactory result of suppressing the coloring property has not been obtained at present (Patent Document 5). In addition, since the silane compound has a low boiling point and volatilizes during processing, there is a problem that workability is deteriorated, and desired performance is not sufficiently exhibited. Furthermore, such silane compounds are known to be poorly compatible with vinyl chloride-based resins and generally highly toxic. It is not preferable to add a silane compound to a medical material, because the silane compound elutes to possibly cause health damage.

Recently, stabilizers for electron beam irradiation sterilization causing severe degradation are also being developed. For example, as stabilizers for electron beam irradiation sterilization, β-diketone, hydrotalcite, alkyl or aryl aminocrotonate, zinc acetylacetonate, zinc dehydroacetate, thioalcohol compound and the like are reported (Patent Documents 6 to 9). However, none of these compounds can be said to have a sufficient effect, and it is difficult to suppress the coloring and the reduction in transparency, especially in electron beam irradiation sterilization causing severe degradation. In addition, recent studies have reported that coloring can be suppressed by blending a silane compound as a radiation-resistant agent, and that 3-mercaptopropionic acid ester is effective even in electron beam irradiation sterilization (Patent Documents 10 and 11). However, satisfactory results have not yet been obtained at present.

Prior Art Document

Patent Documents

Patent Document 1: JP-A-S59-164066
Patent Document 2: JP-A-2006-239026
Patent Document 3: JP-A-H8-24329
Patent Document 4: JP-A-2008-169332
Patent Document 5: JP-A-2015-028116
Patent Document 6: JP-A-H2-263853
Patent Document 7: JP-A-2000-273259
Patent Document 8: WO2015/129534
Patent Document 9: JP-A-H2-222436
Patent Document 10: JP-A-2013-100549
Patent Document 11: JP-A-2012-57099


SUMMARY OF THE INVENTION

Problems to be Solved by the Invention

An object of the present invention is to provide a method capable of solving the above problems and improving the visibility of contents of a material comprising a vinyl chloride-based resin molded body that has been subjected to electron beam irradiation sterilization, as well as to provide a medical material in which the visibility of contents after

electron beam irradiation sterilization has been improved by blending a specific stabilizer. Further, another object of the present invention is to provide a stabilizer for improving the visibility of contents of a material comprising a vinyl chloride-based resin molded body after electron beam irradiation sterilization, and to provide a medical material that comprises a vinyl chloride-based resin molded body containing the stabilizer and has been subjected to electron beam irradiation sterilization. A further object of the present invention is to provide a vinyl chloride-based resin composition used as a raw material of a medical material suitable for radiation sterilization in which coloring after radiation sterilization is significantly suppressed; a vinyl chloride-based resin molded body for a medical material comprising the vinyl chloride-based resin composition; and a sterilization processing method for the vinyl chloride-based resin molded body by radiation irradiation.

MEANS FOR SOLVING THE PROBLEMS

The present inventors have conducted intensive studies to solve the above-mentioned problems, and as a result, have found that the visibility of the contents of a material comprising a vinyl chloride-based resin molded body after electron beam irradiation sterilization is greatly improved by blending a stabilizer comprising a 4-cyclohexene-1,2-dicarboxylic acid diester and/or a 4,5-epoxycyclohexane-1,2-dicarboxylic acid diester. The present invention has been completed based on these findings.

Further, the present inventors have conducted intensive studies to solve the above-mentioned problems, and as a result, have found that a vinyl chloride-based resin composition that can significantly suppress the coloring of a medical material after radiation sterilization can be obtained by blending (a) a 4-cyclohexene-1,2-dicarboxylic acid diester and/or a 4,5-epoxycyclohexane-1,2-dicarboxylic acid diester and (b) at least one selected from the group consisting of a β-diketone compound and a metal salt thereof. The present invention has been completed based on these findings.

That is, the present invention provides a stabilizer containing a 4-cyclohexene-1,2-dicarboxylic acid diester and/or a 4,5-epoxycyclohexane-1,2-dicarboxylic acid diester shown below for improving the visibility of the contents of a material containing a vinyl chloride-based resin molded body after sterilization by electron beam irradiation; a vinyl chloride-based resin composition containing the stabilizer; a medical material having an improved content visibility after electron beam irradiation sterilization and containing a vinyl chloride-based resin molded body using the vinyl chloride-based resin composition as a raw material; and a method for improving the content visibility of a medical material sterilized by electron beam irradiation, the method being characterized by including the stabilizer.

The present invention relates to a method for improving visibility of contents of a material containing a vinyl chloride-based resin molded body that has been subjected to electron beam irradiation sterilization, wherein the vinyl chloride-based resin molded body contains 5 to 100 parts by weight of a 4-cyclohexene-1,2-dicarboxylic acid diester and/or a 4,5-epoxycyclohexane-1,2-dicarboxylic acid diester with respect to 100 parts by weight of a vinyl chloride-based resin.

It is preferable that the 4-cyclohexene-1,2-dicarboxylic acid diester is a compound represented by the following general formula (1):

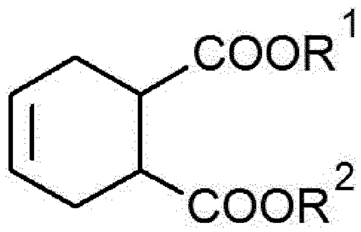

(1)

wherein $R^1$ and $R^2$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms.

It is preferable that the 4,5-epoxycyclohexane-1,2-dicarboxylic acid diester is a compound represented by the following general formula (2):

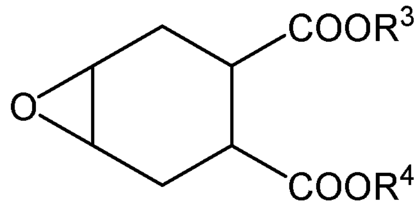

(2)

wherein $R^3$ and $R^4$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms.

In the vinyl chloride-based resin molded body, the rise in the haze value calculated by the following equation is preferably 6 or less:

$$\text{Rise in haze value} = X - Y$$

wherein X is a haze value of a vinyl chloride-based resin molded body after irradiating with an electron beam at a dose of 50 kGy and then allowed to stand under a constant temperature and humidity condition of 25°C and 60% relative humidity for 10 days, and Y is a haze value of the vinyl chloride-based resin molded body before electron beam irradiation.

Also, the present invention relates to a vinyl chloride-based resin composition for a medical material having improved visibility of contents after electron beam irradiation sterilization, the vinyl chloride-based resin composition containing 100 parts by weight of a vinyl chloride-based resin and 5 to 100 parts by weight of a 4-cyclohexene-1,2-dicarboxylic acid diester and/or a 4,5-epoxycyclohexane-1,2-dicarboxylic acid diester.

It is preferable that the vinyl chloride-based resin composition further contains 0.01 to 1.0 part by weight of at least one selected from the group consisting of a β-diketone compound and a metal salt thereof with respect to 100 parts by weight of the vinyl chloride-based resin.

The β-diketone compound and its metal salt are each preferably at least one compound selected from the group consisting of zinc dehydroacetate, calcium acetylacetonate, zinc acetylacetonate, and magnesium acetylacetonate.

The vinyl chloride-based resin composition further contains preferably 0.1 to 30 parts by weight of an epoxy compound other than the 4,5-epoxycyclohexane-1,2-dicarboxylic acid diester with respect to 100 parts by weight of the vinyl chloride-based resin.

The epoxy compound is preferably an epoxidized soybean oil.

Also, the present invention relates to a stabilizer for improving visibility of contents of a material containing a vinyl chloride-based resin molded body after electron beam irradiation sterilization, the stabilizer comprising a 4-cyclohexene-1,2-dicarboxylic acid diester represented by the following general formula (1):

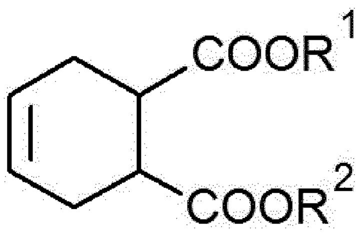

(1)

wherein $R^1$ and $R^2$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms

and/or a 4,5-epoxycyclohexane-1,2-dicarboxylic acid diester represented by the following general formula (2):

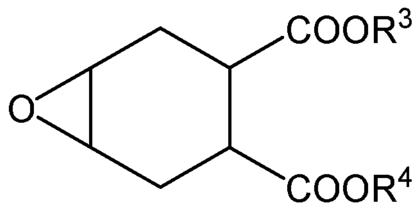

(2)

wherein $R^3$ and $R^4$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms.

Also, the present invention relates to a vinyl chloride-based resin molded body containing the stabilizer.

It is preferable that the vinyl chloride-based resin molded body contains 5 to 100 parts by weight of the stabilizer with respect to 100 parts by weight of a vinyl chloride-based resin.

It is preferable that the vinyl chloride-based resin molded body further contains 0.01 to 1.0 part by weight of at least one selected from a group consisting of a β-diketone compound and a metal salt thereof with respect to 100 parts by weight of a vinyl chloride-based resin.

It is preferable that the β-diketone compound and the metal salt thereof are at least one compound selected from a group consisting of zinc dehydroacetate, calcium acetylacetonate, zinc acetylacetonate, and magnesium acetylacetonate.

It is preferable that the vinyl chloride-based resin molded body further contains 0.1 to 30 parts by weight of an epoxy compound other than the 4,5-epoxycyclohexane-1,2-dicarboxylic acid diester represented by the general formula (2) with respect to 100 parts by weight of a vinyl chloride-based resin.

The epoxy compound is preferably an epoxidized soybean oil.

In the vinyl chloride-based resin molded body, the rise in the haze value calculated by the following equation is preferably 6 or less:

$$\text{Rise in haze value} = X - Y$$

wherein X is a haze value of a vinyl chloride-based resin molded body after irradiating with an electron beam at a dose of 50 kGy and then allowed to stand under a constant temperature and humidity condition of 25°C and 60% relative humidity for 10 days, and Y is a haze value of the vinyl chloride-based resin molded body before electron beam irradiation.

Also, the present invention relates to a medical material sterilized by electron beam irradiation, containing the vinyl chloride-based resin molded body.

Also, the present invention relates to a vinyl chloride-based resin composition that is a raw material for a medical material sterilized by irradiation with radiation, the vinyl chloride-based resin composition containing, with respect to 100 parts by weight of a vinyl chloride-based resin:

(a) 5 to 100 parts by weight of a 4-cyclohexene-1,2-dicarboxylic acid diester represented by a following general formula (1):

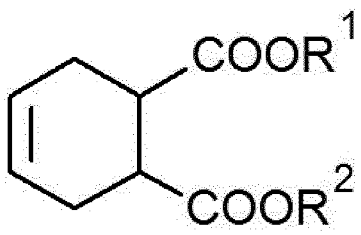

(1)

wherein $R^1$ and $R^2$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms

and/or a 4,5-epoxycyclohexane-1,2-dicarboxylic acid diester represented by the following general formula (2):

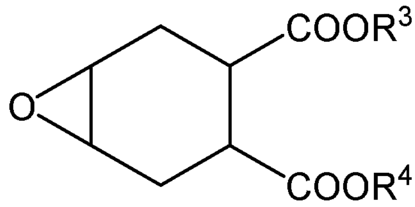

(2)

wherein $R^3$ and $R^4$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms; and

(b) 0.01 to 1.0 part by weight of at least one selected from a group consisting of a β-diketone compound and a metal salt thereof.

The β-diketone compound and its metal salt are each preferably at least one compound selected from the group consisting of zinc dehydroacetate, calcium acetylacetonate, zinc acetylacetonate, and magnesium acetylacetonate.

The vinyl chloride-based resin composition further contains preferably 0.1 to 30 parts by weight of an epoxy compound (c) other than the 4,5-epoxycyclohexane-1,2-dicarboxylic acid diester represented by the general formula (2).

The epoxy compound (c) is preferably an epoxidized soybean oil.

It is preferable that the radiation is an electron beam.

Also, the present invention relates to a vinyl chloride-based resin molded body for a medical material, the vinyl chloride-based resin molded body being obtained from the vinyl chloride-based resin composition.

In the vinyl chloride-based resin molded body for a medical material, the rise in the haze value calculated by the following equation is preferably 6 or less:

$$\text{Rise in haze value} = X - Y$$

wherein X is a haze value of a vinyl chloride-based resin molded body after irradiating with an electron beam at a dose of 50 kGy and then allowed to stand under a constant temperature and humidity condition of 25°C and 60% relative humidity for 10 days, and Y is a haze value of the vinyl chloride-based resin molded body before electron beam irradiation.

Also, the present invention relates to a sterilization processing method by radiation irradiation of a vinyl chloride-based resin molded body for a medical material, the vinyl chloride-based resin molded body being obtained from a vinyl chloride-based resin composition, wherein the vinyl chloride-based resin composition contains, with respect to 100 parts by weight of a vinyl chloride-based resin:

(a) 5 to 100 parts by weight of a 4-cyclohexene-1,2-dicarboxylic acid diester represented by the following general formula (1):

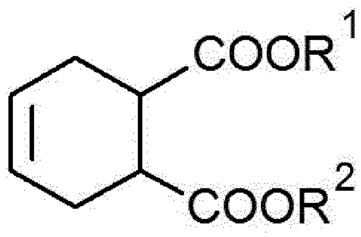

(1)

wherein $R^1$ and $R^2$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms

and/or a 4,5-epoxycyclohexane-1,2-dicarboxylic acid diester represented by the following general formula (2):

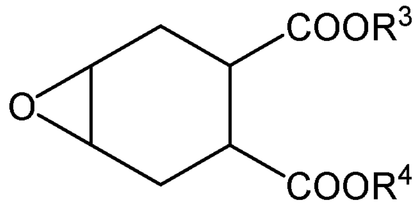

(2)

wherein $R^3$ and $R^4$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms; and

(b) 0.01 to 1.0 part by weight of at least one selected from the group consisting of a β-diketone compound and a metal salt thereof.

The β-diketone compound and its metal salt are each preferably at least one compound selected from the group consisting of zinc dehydroacetate, calcium acetylacetonate, zinc acetylacetonate, and magnesium acetylacetonate.

The vinyl chloride-based resin composition further contains preferably 0.1 to 30 parts by weight of an epoxy compound (c) other than the 4,5-epoxycyclohexane-1,2-dicarboxylic acid diester represented by the general formula (2).

The epoxy compound (c) is preferably an epoxidized soybean oil.

It is preferable that the radiation is an electron beam.

In the vinyl chloride-based resin molded body for a medical material, the rise in the haze value calculated by the following equation is preferably 6 or less:

$$\text{Rise in haze value} = X - Y$$

wherein X is a haze value of a vinyl chloride-based resin molded body after irradiating with an electron beam at a dose of 50 kGy and then allowed to stand under a constant temperature and humidity condition of 25°C and 60% relative humidity for 10 days, and Y is a haze value of the vinyl chloride-based resin molded body before electron beam irradiation.

## EFFECT OF THE INVENTION

By using the stabilizer of the present invention, it is possible to significantly suppress the temporal deterioration of transparency and coloring after sterilization by electron beam irradiation of a material comprising a vinyl chloride-based resin molded body, and as a result, particularly in medical materials, the visibility of the contents after electron beam irradiation sterilization is greatly improved, so that such improvement can greatly help prevent troubles such as medical malpractice.

In the present invention, good visibility of the contents means that, as described above, the transparency necessary for grasping the volume and the flow rate of the contents and the low coloring property necessary for identifying the contents are satisfied at the same time. In the present invention, the haze value is used as an index of transparency, and the smaller the haze value, the better the transparency as well as the better the visibility of the contents. Regarding the coloring property, the yellow index is used as an index, and the smaller the value of the yellow index, the less the coloring as well as the more excellent the visibility of the contents.

Further, by using the vinyl chloride-based resin composition of the present invention, it is possible to obtain a medical material in which coloring over time after radiation irradiation sterilization has been significantly suppressed, which can greatly help to prevent troubles such as medical malpractice.

Further, as described above, the medical material of the present invention satisfies the transparency and the low coloring property necessary for grasping the volume and the flow rate of the contents and has very little change with time.

## MODE FOR CARRYING OUT THE INVENTION

<Stabilizer>

The stabilizer of the present invention contains a 4-cyclohexene-1,2-dicarboxylic acid diester and/or a 4,5-epoxycyclohexane-1,2-dicarboxylic acid diester. It should be noted that the 4-cyclohexene-1,2-dicarboxylic acid diester

and the 4,5-epoxycyclohexane-1,2-dicarboxylic acid diester alone exhibit a function as a plasticizer as well as a function as a stabilizer. The 4-cyclohexene-1,2-dicarboxylic acid diester is preferably a compound having a structure represented by the following general formula (1):

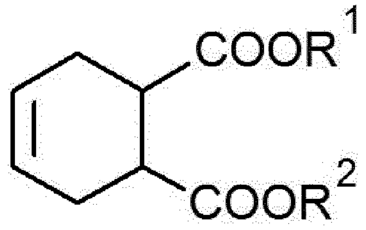

(1)

wherein $R^1$ and $R^2$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms, preferably 8 to 11 carbon atoms.

The 4-cyclohexene-1,2-dicarboxylic acid diester is not particularly limited by its production method so long as the ester satisfies the objective performance of the present invention. For example, the 4-cyclohexene-1,2-dicarboxylic acid diester may be obtained easily by subjecting a 4-cyclohexene-1,2-dicarboxylic acid or its anhydride and a saturated aliphatic alcohol having a specific structure to esterification according to a conventional method, preferably in an atmosphere of an inert gas such as nitrogen in the presence or absence of a catalyst. Also, depending on the kind of the saturated aliphatic alcohol, esterification with a lower alcohol having about 1 to 6 carbon atoms is performed in advance, and after addition of the saturated aliphatic alcohol thereto, transesterification is performed preferably under an inert atmosphere such as nitrogen in the presence or absence of a catalyst, thereby to easily obtain the 4-cyclohexene-1,2-dicarboxylic acid diester.

The 4,5-epoxycyclohexane-1,2-dicarboxylic acid diester has an oxirane oxygen preferably in a range of 1 to 5% by weight, more preferably in a range of 2 to 4% by weight, as measured according to The JOCS Standard Methods for the Analysis of Fats, Oils and Related Materials 2.3.7.1-2013 "Oxirane Oxygen Determination Method (Part 1)". Further, the 4,5-epoxycyclohexane-1,2-dicarboxylic acid diester is preferably a compound having a structure represented by the following general formula (2):

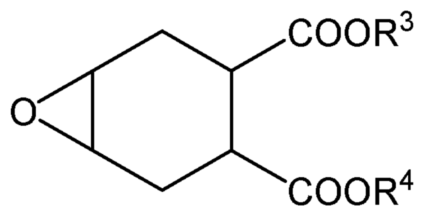

(2)

wherein $R^3$ and $R^4$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms, preferably 8 to 11 carbon atoms.

Generally, epoxy compounds have cytotoxicity, so there is a limit to the amount of epoxy compounds that can be used in the field of medical materials. In general, cytotoxicity tends to increase as the number of epoxy groups in a compound becomes increased. For example, a conventionally used epoxidized soybean oil having a larger amount of epoxy groups in the compound than the compound of the present invention could not be added in an increased amount due to concerns about cytotoxicity as described above.

On the other hand, the effect as a stabilizer tends to show an excellent effect as the number of epoxy groups in the compound becomes larger (that is, as the content of oxirane oxygen becomes higher). For example, reducing the number of epoxy groups in epoxidized vegetable oils such as epoxidized soybean oil results in a reduction in the effect as a stabilizer, and when the number of epoxy groups in the compound is reduced, the blending amount of the stabilizer must be increased to obtain the same effect, which is completely meaningless.

On the other hand, the compound of the present invention is characterized greatly by having a high effect as a stabilizer despite the small number of epoxy groups in the compound, though the reason is not clear. Since the number of epoxy groups in the compound is small and there is little concern about cytotoxicity, it is possible to increase the blending amount of the compound of the present invention even under more severe conditions, and as a result, it is possible to maintain the effect of such a compound as a stabilizer.

The number of epoxy groups in the compound can be usually compared by using the content of oxirane oxygen

measured in accordance with The JOCS Standard Methods for the Analysis of Fats, Oils and Related Materials 2.3.7.1-2013 "Oxirane Oxygen Determination Method (Part 1)". That is, in the case of the compound of the present invention, since the content of oxirane oxygen is in the range of 1 to 5% by weight, excellent visibility of the contents, which is the object of the present invention, is exhibited, and such a compound can be used for medical materials without concerns about cytotoxicity.

The 4,5-epoxycyclohexane-1,2-dicarboxylic acid diester is not particularly limited by its production method so long as it satisfies the object of the present invention. For example, esterification between a 4-cyclohexene-1,2-dicarboxylic acid or its anhydride and a saturated aliphatic alcohol having a specific structure is performed, and the resulting 4-cyclohexene-1,2-dicarboxylic acid diester (intermediate raw material) is epoxidized under predetermined conditions, thereby to easily obtain the 4,5-epoxycyclohexane-1,2-dicarboxylic acid. The 4-cyclohexene-1,2-dicarboxylic acid diester can be also obtained by epoxidizing a 4-cyclohexene-1,2-dicarboxylic acid or its anhydride and esterifying the 4,5-epoxycyclohexane-1,2-dicarboxylic acid or its anhydride with a saturated aliphatic alcohol having a specific structure. Further, depending on the kind of the saturated aliphatic alcohol, there is also a method in which after esterification with a lower alcohol having about 1 to 6 carbon atoms in advance, the saturated aliphatic alcohol described above is added, so that transesterification is performed to obtain the 4,5-epoxycyclohexane-1,2-dicarboxylic acid diester. From the viewpoint of practicality such as simplicity, the method of epoxidation after esterification is most preferable.

[Saturated Aliphatic Alcohol]

The saturated aliphatic alcohol which is the raw material of the 4-cyclohexene-1,2-dicarboxylic acid diester and 4,5-epoxycyclohexane-1,2-dicarboxylic acid diester of the present invention is a linear or branched-chain saturated aliphatic alcohols having preferably 7 to 13 carbon atoms, more preferably 8 to 11 carbon atoms. By using a saturated aliphatic alcohol having 7 to 13 carbon atoms, there is less concern about problems such as volatility and problems such as bleeding, and this is recommended as a preferable embodiment.

More specifically, as the saturated aliphatic alcohol, for example, (i) a linear or branched-chain saturated aliphatic alcohol mainly having 8 carbon atoms is mentioned as a preferred embodiment. Further, when more excellent heat resistance and cold resistance are required, there are recommended embodiments of (ii) a linear or branched-chain saturated aliphatic alcohol having 7 to 13 carbon atoms in which a saturated aliphatic alcohol having 9 carbon atoms is the main component, and the ratio of the saturated aliphatic alcohol having 9 carbon atoms as the main component is preferably at least 60% by weight (60 to 100% by weight), more preferably at least 70% by weight (70 to 100% by weight), particularly preferably at least 80% by weight (80 to 100% by weight), in the linear or branched-chain saturated aliphatic alcohol having 7 to 13 carbon atoms, and (iii) a saturated aliphatic alcohol comprising a linear or branched-chain saturated aliphatic alcohol having mainly 9 to 11 carbon atoms wherein the ratio (molar ratio) of a saturated aliphatic alcohol having 9 carbon atoms/a saturated aliphatic alcohol having 10 carbon atoms/a saturated aliphatic alcohol having 11 carbon atoms is in the range of 10 to 25/35 to 50/30 to 45.

In addition, the above-mentioned "as a main component" means that the ratio in the saturated aliphatic alcohol is 60% by weight or more, preferably 70% by weight or more, more preferably 80% by weight or more. The term "mainly" means that the ratio in the saturated aliphatic alcohol is 90% by weight or more, preferably 95% by weight or more.

In the recommended embodiments of the above (ii) or (iii), the saturated aliphatic alcohol according to the present invention is recommended to have a linear chain ratio of the saturated aliphatic alcohol of preferably 55% by weight or more, more preferably 60% by weight or more, still more preferably 70% by weight or more, particularly preferably 75 to 98% by weight.

In addition, the content of the linear-chain saturated aliphatic alcohol having 9 carbon atoms in the saturated aliphatic alcohol in the recommended embodiment of (ii) is in the range of preferably 60% by weight or more, more preferably 70% by weight or more, particularly preferably 75 to 98% by weight, in the saturated aliphatic alcohol, and the content of the branched-chain saturated aliphatic alcohol having 9 carbon atoms (e.g., 2-methyloctanol, etc.) in the saturated aliphatic alcohol is preferably 40% by weight or less, more preferably 30% by weight or less, particularly preferably 2 to 25% by weight.

As the details of the embodiments of the linear or branched-chain saturated aliphatic alcohol having 7 to 13 carbon atoms, a first preferred embodiment is that the saturated aliphatic alcohol is a linear or branched-chain saturated aliphatic alcohol having mainly 8 carbon atoms. Specifically, commercially available n-octanol, isooctanol, 2-ethylhexanol and the like can be used.

As the details of the embodiments of the linear or branched-chain saturated aliphatic alcohol having 7 to 13 carbon atoms, a second preferred embodiment is that the saturated aliphatic alcohol comprises a saturated aliphatic alcohol having 9 carbon atoms as a main component (preferably 60% by weight or more) and contains 60% by weight or more of a linear-chain saturated aliphatic alcohol having 9 carbon atoms and 40% by weight or less of a branched-chain saturated aliphatic alcohol having 9 carbon atoms, and has a linear chain ratio of 60% by weight or more. A more preferred embodiment is that the linear or branched-chain saturated aliphatic alcohol having 7 to 13 carbon atoms

comprises a saturated aliphatic alcohol having 9 carbon atoms as a main component (preferably 70% by weight or more) and contains 70% by weight or more of a linear-chain saturated aliphatic alcohol having 9 carbon atoms and 30% by weight or less of a branched-chain saturated aliphatic alcohol having 9 carbon atoms, and has a linear chain ratio of 70% by weight or more. A particularly preferred embodiment is that the linear or branched-chain saturated aliphatic alcohol having 7 to 13 carbon atoms comprises a saturated aliphatic alcohol having 9 carbon atoms as a main component (preferably 80% by weight or more) and contains 75 to 98% by weight of a linear-chain saturated aliphatic alcohol having 9 carbon atoms and 2 to 25% by weight of a branched-chain saturated aliphatic alcohol having 9 carbon atoms, and has a linear chain ratio of 75 to 98% by weight.

As the details of the embodiments of the linear or branched-chain saturated aliphatic alcohol having 7 to 13 carbon atoms, a third preferred embodiment is that the saturated aliphatic alcohol is a mixture of saturated aliphatic alcohols each having mainly 9 to 11 carbon atoms, in which the ratio (molar ratio) of each of the alcohols having 9, 10, and 11 carbon atoms is in the range of 10 to 25/35 to 50/30 to 45 and the linear chain ratio is 55% by weight or more, more preferably 60% by weight or more, still more preferably 70% by weight or more, and particularly preferably 75 to 98% by weight.

In the present specification, the linear chain ratio of the linear or branched-chain saturated aliphatic alcohol having 7 to 13 carbon atoms is a ratio (weight ratio) of a linear-chain alcohol in the saturated aliphatic alcohol. Specifically, such a ratio can be determined by an analytical method using gas chromatography.

Specific examples (commercially available products) of the saturated aliphatic alcohol comprising a saturated aliphatic alcohol having 9 carbon atoms as a main component include LINEVOL 9 (trade name) which is a mixture of a linear-chain nonanol of about 80% by weight or more and a branched-chain nonanol of about 20% by weight or less and is manufactured by Shell Chemicals, Inc.; n-nonanol which is a mixture of about 90% by weight or more of a linear-chain nonanol and about 10% by weight or less of a branched-chain nonanol and is manufactured by Oxea GmbH; and the like. Specific examples (commercially available products) of saturated aliphatic alcohols, which are mainly a mixture of saturated aliphatic alcohols each having 9 to 11 carbon atoms, include a LINEVOL 911 (trade name) which contains a linear or branched-chain nonanol, decanol, and undecanol in an amount of 18% by weight, 42% by weight, and 38% by weight, respectively and is manufactured by Shell Chemicals, Inc.

The saturated aliphatic alcohol having 7 to 13 carbon atoms used in the present invention can be produced by a method including, for example, (1) a step of producing an aldehyde having 7 to 13 carbon atoms by a hydroformylation reaction of an $\alpha$-olefin having 6 to 12 carbon atoms, carbon monoxide, and hydrogen and (2) a step of hydrogenating the aldehyde having 7 to 13 carbon atoms to reduce to an alcohol. Further, the saturated aliphatic alcohol having 7 to 13 carbon atoms can be also produced by a method including (I) a step of producing an aldehyde having 3 to 6 carbon atoms by a hydroformylation reaction of an $\alpha$-olefin having 2 to 5 carbon atoms, carbon monoxide and hydrogen and (II) a step of performing an aldol reaction using the aldehyde having 3 to 6 carbon atoms to obtain an aldehyde having 7 to 13 carbon atoms and then hydrogenating the aldehyde having 7 to 13 carbon atoms to reduce to an alcohol.

In the hydroformylation reaction of the steps (1) and (I), for example, 1-octene, carbon monoxide and hydrogen are reacted in the presence of a cobalt catalyst or a rhodium catalyst to be able to produce an aldehyde having 9 carbon atoms.

The hydrogenation of the step (2) can be performed under hydrogen pressure in the presence of, for example, a noble metal catalyst such as a nickel catalyst or a palladium catalyst, thereby to be able to reduce the aldehyde having 9 carbon atoms to an alcohol.

The aldol reaction and hydrogenation in the step (II) are carried out, for example, by subjecting butyraldehyde to aldol condensation using a catalyst such as potassium hydroxide or sodium hydroxide to produce 2-ethyl-2-hexenal, and then hydrogenating the 2-ethyl-2-hexenal under hydrogen pressure in the presence of a noble metal catalyst such as a nickel catalyst or a palladium catalyst to be able to reduce the aldehyde to an alcohol.

For the 4-cyclohexene-1,2-dicarboxylic acid diester and 4,5-epoxycyclohexane-1,2-dicarboxylic acid diester, the saturated aliphatic alcohol may be appropriately selected according to various requirements in various medical materials. For example, when plasticization efficiency and flexibility are required or when a functional balance is required, the linear or branched-chain saturated aliphatic alcohol having mainly 8 carbon atoms in the above-mentioned (i) is preferably 2-ethylhexanol. Similarly, the saturated aliphatic alcohols having a good functional balance include a branched-chain alcohol such as 3,5,5-trimethylhexanol, isononyl alcohol, and isodecyl alcohol, and these also have good migration resistance. When a saturated aliphatic alcohol comprising mainly the saturated aliphatic alcohol having 9 carbon atoms described in (ii) above is used as the saturated aliphatic alcohol, such an aliphatic alcohol is also excellent in heat resistance and volatility and thus is suitable, for example, for medical devices used in combination with other sterilization methods such as steam sterilization. When a saturated aliphatic alcohol, which is a mixture of the saturated aliphatic alcohols having mainly 9 to 11 carbon atoms of the above (iii), is used as the saturated aliphatic alcohol, not only its heat resistance and volatility are excellent, but also its migration resistance is excellent, so that such a saturated aliphatic alcohol can be used with confidence in medical material applications where durability is required.

[Esterification Reaction]

In the production of the 4-cyclohexene-1,2-dicarboxylic acid diester of the present invention, when the esterification reaction of the saturated aliphatic alcohol with a 4-cyclohexene-1,2-dicarboxylic acid or an acid anhydride thereof is carried out, it is recommended to use the saturated aliphatic alcohol in an amount of, for example, preferably 2.05 to 4.00 moles, more preferably 2.10 to 3.00 moles, particularly preferably 2.15 to 2.50 moles, with respect to 1 mole of the 4-cyclohexene-1,2-dicarboxylic acid or an anhydride thereof.

Further, in the production of the 4-cyclohexene-1,2-dicarboxylic acid diester which is an intermediate material of the 4,5-epoxycyclohexane-1,2-dicarboxylic acid diester of the present invention, when performing an esterification reaction between the saturated aliphatic alcohol and the 4-cyclohexene-1,2-dicarboxylic acid or an acid anhydride thereof, it is recommended to use the saturated aliphatic alcohol in an amount of, for example, preferably 2.00 to 5.00 moles, more preferably 2.01 to 3.00 moles, especially 2.02 to 2.50 moles, with respect to 1 mole of the 4-cyclohexene-1,2-dicarboxylic acid or an acid anhydride thereof.

In the case of using a catalyst for the esterification reaction, examples of the catalyst include a mineral acid, an organic acid, a Lewis acid, and the like. More specifically, examples of the mineral acid include sulfuric acid, hydrochloric acid, phosphoric acid, and the like. Examples of the organic acid include p-toluenesulfonic acid, methanesulfonic acid, and the like. Examples of the Lewis acid include an aluminum derivative, a tin derivative, a titanium derivative, a lead derivative, a zinc derivative, and the like. One or two or more kinds of these acids can be used singly or in combination.

Among them, p-toluenesulfonic acid, tetraalkyl titanate having 3 to 8 carbon atoms, titanium oxide, titanium hydroxide, tin fatty acid having 3 to 12 carbon atoms, tin oxide, tin hydroxide, zinc oxide, zinc hydroxide, lead oxide, lead hydroxide, aluminum oxide, and aluminum hydroxide are particularly preferable. The amount of the catalyst to be used is recommended to be preferably from 0.01% by weight to 5.0% by weight, more preferably from 0.02% by weight to 4.0% by weight, particularly preferably from 0.03% by weight to 3.0% by weight, with respect to the total weight of the acid component and the alcohol component which are each a raw material for synthesizing the ester compound.

The esterification temperature is exemplified by 100°C to 230°C, and the esterification reaction is usually completed within 3 hours to 30 hours.

The 4-cyclohexene-1,2-dicarboxylic acid or its acid anhydride, which is a raw material for the 4-cyclohexene-1,2-dicarboxylic acid diester and 4,5-epoxycyclohexane-1,2-dicarboxylic acid diester of the present invention, is not particularly limited, and those produced by known methods, commercially available products, available reagents, and the like can be used. For example, RIKACID TH (trade name, manufactured by New Japan Chemical Co., Ltd.) and the like are exemplified as a commercially available product. The 4-cyclohexene-1,2-dicarboxylic anhydride is usually obtained by Diels-Alder reaction between maleic anhydride and 1,3-butadiene. From the viewpoint of esterification reaction, 4-cyclohexene-1,2-dicarboxylic anhydride is recommended to be used as a raw material.

In the esterification reaction, a water entraining agent such as benzene, toluene, xylene, and cyclohexane can be used to accelerate the distillation of water produced by the reaction.

When an oxygen-containing organic compound such as an oxide, a peroxide, and a carbonyl compound is produced due to oxidative deterioration of the raw material, the produced ester and the organic solvent (water entraining agent) during the esterification reaction, the oxygen-containing organic compound exerts an adverse effect on heat resistance, weather resistance, and the like. Thus, it is desirable to carry out the esterification reaction in an inert gas atmosphere such as nitrogen gas or in an inert gas stream at normal pressure or under reduced pressure. After completion of the esterification reaction, it is recommended to distill off the excess raw material under reduced pressure or atmospheric pressure.

The 4-cyclohexene-1,2-dicarboxylic acid diester obtained by the esterification method may be subsequently purified by base treatment (neutralization treatment), water washing treatment, liquid-liquid extraction, distillation (depressurization, dehydration treatment), adsorption purification treatment and the like as needed.

The base used in the base treatment is not particularly limited as long as the base is a basic compound, and examples thereof include sodium hydroxide, sodium carbonate, and the like.

Examples of the adsorbent used in the adsorption purification treatment include activated carbon, activated clay, activated alumina, hydrotalcite, silica gel, silica alumina, zeolite, magnesia, calcia, diatomaceous earth, and the like. These adsorbents can be used singly or in appropriate combination of two or more kinds thereof. The above processing may be carried out at normal temperature, but may also be carried out by heating to about 40 to 100°C or about 40 to 90°C.

[Epoxidation Reaction]

The epoxidation reaction as used herein means an epoxidation reaction of an unsaturated bond in the 4-cyclohexene-1,2-dicarboxylic acid diester which is an intermediate material for obtaining the 4,5-epoxycyclohexane-1,2-dicarboxylic acid diester of the present invention and is usually carried out easily by using a well-known epoxidation

reaction described in "Yuki Gosei Kagaku (Synthetic Organic Chemistry) Vol. 23, No. 7, pp 612-619 (1985)" or the like. For example, there are exemplified by (i) a method in which an organic peracid such as peracetic acid and performic acid is used as an epoxidizing agent and (ii) a method in which hydrogen peroxide is used as an epoxidizing agent.

More specifically, in the case of the method (i), the reaction is completed, for example, by adding peracetic acid obtained by reacting hydrogen peroxide with acetic anhydride or acetic acid in the presence of a strong acid such as sulfuric acid as a catalyst to the 4-cyclohexene-1,2-dicarboxylic acid diester, stirring the mixture at 20 to 30°C for several hours, gradually raising the temperature to reach the temperature of 50 to 60°C, and maintaining the temperature for 2 to 3 hours. As the organic peracid, monoperphthalic acid, m-chloroperbenzoic acid, trifluoroperacetic acid and the like can also be used in addition to the above peracids.

In the case of the method (ii), the epoxidation can be carried out, for example, by reacting hydrogen peroxide with the ester in the co-presence of an oxygen carrier such as formic acid and a strong acid catalyst such as sulfuric acid. More specifically, using acetic acid or formic acid in a small amount of 0.5 moles or less and sulfuric acid as a catalyst in a small amount of 0.05 moles or less with respect to 1 mole of hydrogen peroxide, the reaction is carried out by maintaining the mixture at 40 to 70°C for 2 to 15 hours. Thereby, the 4-cyclohexene-1,2-dicarboxylic acid diester can be easily epoxidized. As the catalyst, phosphoric acid, hydrochloric acid, nitric acid, boric acid, or salts thereof are well known in addition to the above catalysts, and sulfonic acid type strongly acidic cation exchange resins, aluminum oxides, and the like are also effective.

The 4,5-epoxycyclohexane-1,2-dicarboxylic acid diester obtained by the epoxidation method may be subsequently purified by liquid-liquid extraction, vacuum distillation, adsorption treatment or the like as needed.

Examples of the adsorbent used in the adsorption treatment include activated carbon, activated clay, activated alumina, hydrotalcite, silica gel, silica alumina, zeolite, magnesia, calcia, diatomaceous earth and the like. These adsorbents can be used singly or in appropriate combination of two or more kinds thereof.

The treatment may be carried out at normal temperature, but such purification may also be carried out by heating to about 40 to 100°C.

<Vinyl Chloride-Based Resin Composition>

The vinyl chloride-based resin composition of the present invention can be obtained by blending a vinyl chloride-based resin with a stabilizer comprising a 4-cyclohexene-1,2-dicarboxylic acid and/or a 4,5-epoxycyclohexane-1,2-dicarboxylic acid diester, or by blending the stabilizer with another plasticizer in combination.

Further, the vinyl chloride-based resin composition of the present invention can be obtained by blending a vinyl chloride-based resin with (a) a 4-cyclohexene-1,2-dicarboxylic acid diester and/or a 4,5-epoxycyclohexane-1,2-dicarboxylic acid diester and (b) at least one selected from the group consisting of a β-diketone compound and a metal salt thereof, or by blending a vinyl chloride-based resin in combination with other plasticizers and other stabilizers.

[(b) β-Diketone Compound and Metal Salt Thereof]

The β-diketone compound and its metal salt function as a stabilizer and suppress coloring. Examples of the β-diketone compound include acetylacetone, triacetylmethane, 2,4,6-heptatrione, butanoylacetylmethane, lauroylacetylmethane, palmitoylacetylmethane, stearoylacetylmethane, phenylacetylacetylmethane, dicyclohexylcarbonylmethane, benzoylformylmethane, benzoylacetylmethane, dibenzoylmethane, octylbenzoylmethane, stearoylbenzoylmethane, bis(4-octylbenzoyl)methane, benzoyldiacetylmethane, 4-methoxybenzoylbenzoylmethane, bis(4-carboxymethyl-benzoyl)methane, 2-carboxymethyl-benzoylacetyloctylmethane, dehydroacetic acid, cyclohexane-1,3-dione, methyl 3,6-dimethyl-2,4-dioxycyclohexane-1-carboxylate, 2-acetylcyclohexanone, dimedone, 2-benzoylcyclohexane, palmitoylbenzoylmethane, distearoylmethane, ethyl acetoacetate, and the like. Examples of metal salts include lithium, sodium, potassium, calcium, zinc, magnesium, and aluminum salts, and the like. Preferred are metal salts of β-diketone compounds, and specific examples include zinc dehydroacetate, calcium acetylacetonate, zinc acetylacetonate, and magnesium acetylacetonate. Particularly preferred are zinc dehydroacetate and zinc acetylacetonate. One of these β-diketone compounds may be used alone, or two or more kinds thereof may be used in combination. At least one selected from the group consisting of a β-diketone compound and a metal salt thereof is blended in an amount of 0.01 to 1.0 part by weight, preferably 0.05 to 0.5 parts by weight, more preferably 0.2 to 0.5 parts by weight, with respect to 100 parts by weight of the vinyl chloride-based resin.

[Vinyl Chloride-Based Resin]

The vinyl chloride-based resin used in the present invention is a homopolymer of vinyl chloride or vinylidene chloride, and a copolymer of vinyl chloride or vinylidene chloride, and the production method thereof is carried out by a conventionally known polymerization method. For example, in the case of a versatile vinyl chloride-based resin, a method

of suspension polymerization in the presence of an oil-soluble polymerization catalyst and the like can be mentioned. In the case of a vinyl chloride paste resin, a method of emulsion polymerization in an aqueous medium in the presence of a water-soluble polymerization catalyst and the like can be mentioned. The degree of polymerization of these vinyl chloride-based resins is usually from 300 to 5000, preferably from 400 to 3500, even more preferably from 700 to 3000. If the degree of polymerization is too low, the heat resistance and the like are deteriorated, whereas if the degree of polymerization is too high, the molding processability tends to decrease.

In the case of a copolymer, examples thereof include copolymers of a vinyl chloride monomer and an $\alpha$-olefin having 2 to 30 carbon atoms (e.g. ethylene, propylene, 1-butene, 1-pentene, 1-hexene, 1-heptene, 1-octene, 1-nonene, 1-decene, 1-undecene, 1-dodecene, 1-tridecene, 1-tetradecene, etc.), acrylic acid or esters thereof, methacrylic acid and esters thereof, maleic acid and esters thereof, a vinyl compound (e.g. vinyl acetate, vinyl propionate, alkyl vinyl ether, etc.), a polyfunctional monomer (e.g. diallyl phthalate, etc.), or a mixture thereof; ethylene-acrylate ester copolymers (e.g. ethylene-ethyl acrylate copolymer, etc.); ethylene-methacrylate ester copolymers; ethylene-vinyl acetate copolymers (EVA); chlorinated polyethylene; butyl rubber; crosslinked acrylic rubber; polyurethane; butadiene-styrene-methyl methacrylate copolymers (MBS); butadiene-acrylonitrile-($\alpha$-methyl)styrene copolymers (ABS); styrene-butadiene copolymers; and graft copolymers obtained by grafting a vinyl chloride monomer to polyethylene, polymethyl methacrylate or a mixture thereof; and the like.

[(c) Epoxy Compound]

It is preferable that the vinyl chloride-based resin composition of the present invention contains an epoxy compound including an epoxidized animal and vegetable oil such as epoxidized soybean oil, epoxidized linseed oil, epoxidized castor oil, and epoxidized fish oil; an epoxidized fatty acid ester such as methyl epoxystearate, butyl epoxystearate, octyl epoxystearate, and epoxidized linseed oil fatty acid butyl ester; and the like other than the 4,5-epoxycyclohexane-1,2-dicarboxylic acid diester. More preferred are epoxidized animal and vegetable oils such as epoxidized soybean oil, epoxidized linseed oil, epoxidized castor oil, epoxidized fish oil, and most preferably epoxidized soybean oil. The blending amount of the epoxy compound is preferably 0.1 to 30 parts by weight, more preferably 1 to 20 parts by weight, still more preferably 5 to 15 parts by weight, particularly preferably 5 to 10 parts by weight, with respect to 100 parts by weight of the vinyl chloride-based resin. The epoxy compound also functions as a plasticizer, but when used in combination with the above-mentioned (a) a 4-cyclohexene-1,2-dicarboxylic acid diester and/or a 4,5-epoxycyclohexane-1,2-dicarboxylic acid diester and (b) at least one selected from the group consisting of $\beta$-diketone compounds and a metal salt thereof, it is possible to further suppress coloring.

[Vinyl Chloride-Based Resin Composition]

The vinyl chloride-based resin composition of the present invention comprises a 4-cyclohexene-1,2-dicarboxylic acid diester and/or a 4,5-epoxycyclohexane-1,2-dicarboxylic acid diester in an amount of 5 to 100 parts by weight, preferably 10 to 70 parts by weight, more preferably 10 to 50 parts by weight, still more preferably 20 to 50 parts by weight, and another plasticizer in an amount of 0 to 100 parts by weight, preferably 0 to 50 parts by weight, more preferably 10 to 30 parts by weight, with respect to 100 parts by weight of the vinyl chloride-based resin. It should be noted that when the 4-cyclohexene-1,2-dicarboxylic acid diester and the 4,5-epoxycyclohexane-1,2-dicarboxylic acid diester are used in combination, the parts by weight described above are the total amount. If the amount of the 4-cyclohexene-1,2-dicarboxylic acid diester and/or the 4,5-epoxycyclohexane-1,2-dicarboxylic acid diester is within the above range, the effect of the present invention, that is, reduction in transparency and coloring due to (electron beam) irradiation sterilization of the medical materials can be suppressed. As a result, a reduction in the visibility of the contents can be suppressed, and the performance as a plasticizer is also exhibited. In addition, the other plasticizers are appropriately adjusted depending on the flexibility required according to the intended use but are generally applicable to almost all medical materials within the above range. It should be noted that the 4-cyclohexene-1,2-dicarboxylic acid diester and the 4,5-epoxycyclohexane-1,2-dicarboxylic acid diester also have a certain plasticizing performance. Accordingly, it is also possible to blend the 4-cyclohexene-1,2-dicarboxylic acid diester or the 4,5-epoxycyclohexane-1,2-dicarboxylic acid diester alone as a stabilizer as well as a plasticizer.

[Other Plasticizers]

The "other plasticizer" in the present invention refers to a plasticizer excluding a 4-cyclohexene-1,2-dicarboxylic acid diester or a compound represented by the above general formula (1), or a plasticizer excluding a 4,5-epoxycyclohexane-1,2-dicarboxylic acid diester or a compound represented by the general formula (2), and a plasticizer generally used for vinyl chloride-based resin can be used alone or in combination of two or more kinds thereof. Specific examples of such known plasticizers include benzoic acid esters (e.g. diethylene glycol dibenzoate, etc.); phthalic acid esters (e.g.

dibutyl phthalate (DBP), di-2-ethylhexyl phthalate (DEHP), diisononyl phthalate (DINP), diisodecyl phthalate (DIDP), diundecyl phthalate (DUP), ditridecyl phthalate (DTDP), bis(2-ethylhexyl) terephthalate (DOTP), bis(2-ethylhexyl) isophthalate (DOIP), etc.); aliphatic dibasic acid esters (e.g. di-2-ethylhexyl adipate (DOA), diisononyl adipate (DINA), diisodecyl adipate (DIDA), di-2-ethylhexyl sebacate (DOS), diisononyl sebacate (DINS), etc.); trimellitic acid esters (e.g. tri-2-ethylhexyl trimellitate (TOTM), triisononyl trimellitate (TINTM), triisodecyl trimellitate (TIDTM), etc.); pyromellitic acid esters (e.g. tetra-2-ethylhexyl pyromellitate (TOPM), etc.); phosphoric acid esters (e.g. tri-2-ethylhexyl phosphate (TOP), tricresyl phosphate (TCP), etc.); alkyl esters of polyhydric alcohols such as pentaerythritol; polyesters having a molecular weight of from 800 to 4000 synthesized by polyesterification of a dibasic acid (e.g. adipic acid, etc.) and a glycol; epoxidized animal and vegetable oils (e.g. epoxidized soybean oil, epoxidized linseed oil, epoxidized castor oil, epoxidized fish oil, etc.); epoxidized fatty acid esters (e.g. methyl epoxystearate, butyl epoxystearate, octyl epoxystearate, epoxidized linseed oil fatty acid butyl ester, etc.); alicyclic dibasic acid esters (e.g. diepoxystearyl epoxyhexahydrophthalate, diisononyl 1,2-cyclohexanedicarboxylate (DINCH), etc.); fatty acid glycol esters (e.g. 1,4-butanediol dicaprate, etc.); citric acid esters (e.g. acetyl tributyl citrate (ATBC), acetyl trihexyl citrate (ATHC), acetyl triethylhexyl citrate (ATEHC), butyryl trihexyl citrate (BTHC), etc.); isosorbide diesters; chlorinated paraffins obtained by chlorinating paraffin wax or n-paraffin; chlorinated fatty acid esters (e.g. chlorinated stearic acid ester, etc.); higher fatty acid esters (e.g. butyl oleate, etc.); and the like. Among these, in the case of blood bags and blood-related medical materials, DEHP tends to be preferably used, and from the viewpoint of recent environmental issues, trimellitic acid esters (e.g. TOTM, etc.) and alicyclic dibasic acid esters (e.g. DINCH, etc.) are preferably used. The blending amount of the plasticizer is appropriately adjusted according to the intended use as described above within a range not impairing the effects of the present invention, and is usually 0 to 100 parts by weight, preferably 0 to 50 parts by weight, more preferably 10 to 30 parts by weight, with respect to 100 parts by weight of the vinyl chloride-based resin.

In the vinyl chloride-based resin composition of the present invention, it is also possible to blend, as required, additives such as stabilizers other than those of the present invention, stabilizing aids, antioxidants (aging inhibitors), ultraviolet absorbers, silane compound-based radiation resistant agents, hindered amine-based light stabilizers, diluents, viscosity reducing agents, thickeners, processing aids, lubricants, antistatic agents, flame retardants, foaming agents, adhesives, and coloring agents.

Stabilizers other than those of the present invention include metal soap compounds such as lithium stearate, magnesium stearate, magnesium laurate, calcium ricinoleate, calcium stearate, barium laurate, barium ricinoleate, barium stearate, zinc octylate, zinc laurate, zinc ricinoleate, and zinc stearate; organic tin-based compounds such as dimethyltin bis-2-ethylhexyl thioglycolate, dibutyltin maleate, dibutyltin bisbutylmaleate, and dibutyltin dilaurate; hydrotalcite, aminocrotonic acid alkyl or aryl esters, thioalcohol compounds, 3-mercaptopropionic acid esters, and antimony mercaptide compounds. In addition, epoxidized vegetable oils such as epoxidized soybean oil, epoxidized linseed oil, and epoxidized castor oil, which are one type of the plasticizers, also have an effect as a stabilizer for suppressing coloring, and can be blended within a range that does not cause problems such as bleeding and cytotoxicity as described above. When such a stabilizer is blended, the blending amount is recommended to be usually about 0.1 to 20 parts by weight with respect to 100 parts by weight of the vinyl chloride-based resin.

From the viewpoint of safety, it is preferable to use at least one selected from organic acid zinc and organic acid calcium among the above stabilizers, and a combination of zinc stearate and calcium stearate is most preferable. The blending amount in total is about 0.1 to 10 parts by weight, preferably 0.2 to 6 parts by weight, with respect to 100 parts by weight of the vinyl chloride-based resin. The blending ratio (weight ratio) of the stabilizer is not particularly limited so long as it is within a range where the effect of stabilization is exhibited, but usually the stabilizer is often used in the range of 5:1 to 1:5.

Examples of the stabilizing aid include phosphite-based compounds such as triphenyl phosphite, monooctyl diphenyl phosphite, and tridecyl phosphite; polyol compounds such as glycerin, sorbitol, pentaerythritol, and polyethylene glycol; perchlorate compounds such as barium perchlorate and sodium perchlorate; hydrotalcite compounds; zeolites; and the like. When a stabilizing aid is blended, the blending amount is recommended to be about 0.1 to 20 parts by weight with respect to 100 parts by weight of the vinyl chloride-based resin.

Examples of the silane compound-based radiation-resistant material include monoalkoxysilane compounds such as trimethylmethoxysilane, trimethylethoxysilane, triethylmethoxysilane, and triethylethoxysilane; dialkoxysilane compounds such as dimethyldimethoxysilane, diethyldimethoxysilane, dimethyldiethoxysilane, diphenyldimethoxysilane, diphenyldiethoxysilane, methylaminoethoxypropyldialkoxysilane, N-(β-aminoethyl)-y-aminopropylmethyldimethoxysilane, γ-glycidoxypropylmethyldimethoxysilane, and γ-methacryloxypropylmethyldimethoxysilane; trialkoxysilane compounds such as methyltrimethoxysilane, methyltriethoxysilane, hexyltrimethoxysilane, phenyltrimethoxysilane, phenyltriethoxysilane, vinyltrimethoxysilane, vinyltriethoxysilane, γ-chloropropyltrimethoxysilane, γ-aminopropyltriethoxysilane, N-(β-aminoethyl)-γ-aminopropyltrimethoxysilane, N-(phenyl)-γ-aminopropyltrimethoxysilane, γ-glycidoxypropyltrimethoxysilane, 3-methacryloxypropyl-trimethoxysilane, 3-methacryloxypropyltriethoxysilane, γ-mercaptopropyltrimethoxysilane, γ-(polyethyleneamino)propyltrimethoxysilane, γ-ureidopropyltriethoxysilane, heptadecafluorodecyl-trimethoxysilane, tridecafluorooctyltrimethoxysilane, vinyltris(β-methoxyethoxy)silane, and β-(3,4-epoxycyclohexyl)ethyltrimethoxysilane; tetraalkoxysilane compounds such as tetramethoxysilane and tetraethoxysilane; acetoxysilane compounds such as vinyltriacetoxysilane; chlorosilane compounds such as trimethylchlorosilane, dimethyldichlorosilane, methyltrichlorosilane, vinyltrichlorosilane, and γ-chloropropylmethyldichlorosilane; organosilane compounds such as triisopropylsilane, triisopropylsilyl acrylate, allyltrimethylsilane, and methyl trimethylsilylacetate; and the like. When blending a radiation resistant agent, the blending amount is recommended to be about 0.1 to 15 parts by weight with respect to 100 parts by weight of the vinyl chloride-based resin.

Examples of the antioxidant include phenol-based compounds (e.g. 2,6-di-tert-butylphenol, tetrakis[methylene-3-(3,5-tert-butyl-4-hydroxyphenol) propionate]methane, 2-hydroxy-4-methoxybenzophenone, etc.); sulfur compounds (e.g. alkyl disulfides, thiodipropionate esters, benzothiazoles, etc.); phosphate-based compounds (e.g. tris(nonylphenyl) phosphite, diphenyl isodecyl phosphite, triphenyl phosphite, tris(2,4-di-t-butylphenyl) phosphite, etc.); organometallic compounds (e.g. zinc dialkyl dithiophosphate, zinc diaryl dithiophosphate, etc.); and the like. When an antioxidant is blended, the blending amount is recommended to be about from 0.2 to 20 parts by weight with respect to 100 parts by weight of the vinyl chloride-based resin.

Examples of the ultraviolet absorber include salicylate-based compounds such as phenyl salicylate and p-tert-butylphenyl salicylate; benzophenone-based compounds such as 2-hydroxy-4-n-octoxybenzophenone and 2-hydroxy-4-methoxybenzophenone; benzotriazole-based compounds such as 5-methyl-1H-benzotriazole, 1-dioctylaminomethylbenzotriazole; cyanoacrylate-based compounds; and the like. When an ultraviolet absorber is blended, the blending amount is recommended to be about from 0.1 to 10 parts by weight with respect to 100 parts by weight of the vinyl chloride-based resin.

Examples of the hindered amine-based light stabilizer include bis(2,2,6,6-tetramethyl-4-piperidyl) sebacate, bis(1,2,2,6,6-pentamethyl-4-piperidyl) sebacate and 1,2,2,6,6-pentamethyl-4-piperidyl sebacate (mixture), bis(1,2,2,6,6-pentamethyl-4-piperidyl)[[3,5-bis(1,1-dimethylethyl)-4-hydroxyphenyl]methyl]butyl malonate, decanedioic acid bis(2,2,6,6-tetramethyl-1(octyloxy)-4-piperidyl)ester, and a reaction product of 1,1-dimethylethyl hydroperoxide and octane, 4-benzoyloxy-2,2,6,6-tetramethylpiperidine, an ester mixture of 2,2,6,6-tetramethyl-4-piperidinol and a higher fatty acid, tetrakis(2,2,6,6-tetramethyl-4-piperidyl)-1,2,3,4-butanetetracarboxylate, tetrakis(1,2,2,6,6-pentamethyl-4-piperidyl)-1,2,3,4-butanetetracarboxylate, a polycondensation product of dimethyl succinate and 4-hydroxy-2,2,6,6-tetramethyl-1-piperidine ethanol, poly[{(6-(1,1,3,3-tetramethylbutyl)amino-1,3,5-triazine-2,4-diyl){(2,2,6,6-tetramethyl-4-piperidyl)imino)hexamethylene{(2,2,6,6-tetramethyl-4-piperidyl)imino}}, a polycondensation product of dibutylamine/1,3,5-triazine/N,N'-bis(2,2,6,6-tetramethyl-4-piperidyl-1,6-hexamethylenediamine and N-(2,2,6,6-tetramethyl-4-piperidyl)butylamine, N,N',N'',N'''-tetrakis-(4,6-bis-(butyl-(N-methyl-2,2,6,6-tetramethylpiperidine-4-yl)amino)-triazine-2-yl)-4,7-diazadecane-1,10-diamine, and the like. When a light stabilizer is blended, the blending amount is recommended to be about from 0.1 to 10 parts by weight with respect to 100 parts by weight of the vinyl chloride-based resin.

Examples of the diluent include 2,2,4-trimethyl-1,3-pentanediol diisobutyrate, and aliphatic or aromatic hydrocarbons having a low boiling point. When blending a diluent, the blending amount is recommended to be about 1 to 50 parts by weight with respect to 100 parts by weight of the vinyl chloride-based resin.

Examples of the viscosity reducing agent include various non-ionic surfactants, sulfosuccinate-based anionic surfactants, silicone-based compounds having surface activity, soybean oil lecithin, monohydric alcohols, glycol ethers, polyethylene glycols, and the like. When blending a viscosity reducing agent, the blending amount is recommended to be about 0.1 to 20 parts by weight with respect to 100 parts by weight of the vinyl chloride-based resin.

Examples of the thickener include synthetic fine powder silica-based thickeners, bentonite-based thickeners, ultra-fine precipitated calcium carbonates, metal soap-based thickeners, hydrogenated castor oil, polyamide wax, polyethylene oxide-based thickeners, vegetable oil-based thickeners, sulfate-based surfactants, non-ionic surfactants, and the like. When blending a thickener, the blending amount is recommended to be about 1 to 50 parts by weight with respect to 100 parts by weight of the vinyl chloride-based resin.

Examples of the processing aid include liquid paraffin, polyethylene wax, stearic acid, stearic acid amide, ethylene bis(stearic acid amide), butyl stearate, and calcium stearate. When a processing aid is blended, the blending amount is recommended to be about from 0.1 to 20 parts by weight with respect to 100 parts by weight of the vinyl chloride-based resin.

Examples of the lubricant include silicone; liquid paraffin; paraffin wax; fatty acid metal salts such as metal stearate and metal laurate; fatty acid amide; fatty acid wax; and higher fatty acid wax. When a lubricant is blended, the blending amount is recommended to be about from 0.1 to 10 parts by weight with respect to 100 parts by weight of the vinyl chloride-based resin.

Examples of the antistatic agent include anionic antistatic agents such as alkyl sulfonate antistatic agents, alkyl ether carboxylic acid antistatic agents, or dialkyl sulfosuccinate antistatic agents; nonionic antistatic agents such as polyethylene glycol derivatives, sorbitan derivatives or diethanolamine derivatives; quaternary ammonium salts such as alkylamide amine antistatic agents or alkyl dimethylbenzyl antistatic agents; cationic antistatic agents such as alkyl pyridinium organic acid salt or hydrochloride; and amphoteric antistatic agents such as alkylbetaine antistatic agents or alkylimidazoline antistatic agents. When an antistatic agent is blended, the blending amount is recommended to be

about from 0.1 to 10 parts by weight with respect to 100 parts by weight of the vinyl chloride-based resin.

Examples of the flame retardant include: inorganic compounds such as aluminum hydroxide, antimony trioxide, magnesium hydroxide, and zinc borate; phosphorus compounds such as cresyl diphenyl phosphate, trischloroethyl phosphate, trischloropropyl phosphate, and trisdichloropropyl phosphate; and halogen compounds such as chlorinated paraffin. When a flame retardant is blended, it is recommended that the blending amount of the flame retardant is about 0.1 to 20 parts by weight with respect to 100 parts by weight of the vinyl chloride-based resin.

Examples of the foaming agent include: organic foaming agents such as azodicarbonamide and oxybisbenzenesulfonyl hydrazide; inorganic foaming agents such as sodium bicarbonate; and the like. When a foaming agent is blended, it is recommended that the blending amount of the foaming agent is about 0.1 to 30 parts by weight with respect to 100 parts by weight of the vinyl chloride-based resin.

Examples of the colorant include those that have an effect of improving transparency, such as phthalocyanine green, cobalt blue, and phthalocyanine blue, and the colorant may be blended in a small amount so as not to impair the visibility in the present invention.

The vinyl chloride-based resin composition according to the present invention can be molded into a powdery, pellet-like, or paste-like vinyl chloride-based resin composition by handling mixing or stir mixing/melt mixing a vinyl chloride-based resin, the stabilizer of the present invention and other plasticizers depending on the intended use, and further various additives as needed, with use of, for example, an agitator such as a pony mixer, a butterfly mixer, a planetary mixer, a dissolver, a twin-screw mixer, a three-roll mill, a mortar mixer, a Henschel mixer, or a Banbury mixer, and a ribbon blender, or a kneader such as a conical twin-screw extruder, a parallel twin-screw extruder, a single screw extruder, a co-kneader type kneader, and a roll kneader.

[Vinyl Chloride-Based Resin Molded Body]

The vinyl chloride-based resin composition according to the present invention can be molded into a vinyl chloride-based resin molded body having a desired shape by molding process using a conventionally known method such as vacuum molding, compression molding, extrusion molding, injection molding, calendar molding, press molding, blow molding, powder molding, spread coating, dip coating, spray coating, paper casting, extrusion coating, gravure printing, screen printing, slush molding, rotational molding, casting, dip molding, and welding.

The shape of the vinyl chloride-based resin molded body is not particularly limited, but may include, for example, a rod shape, a sheet shape, a film shape, a plate shape, a cylindrical shape, a circular shape, an elliptical shape or the like or a special shape such as a star shape and a polygonal shape.

The vinyl chloride-based resin molded body can be used as a material for various uses, and in particular, can be used preferably as a medical material, for example, tubes (e.g. chest tubes, dialysis tubes, artificial respiration tubes, endotracheal tubes, respiration tubes, nutrition feeding tubes, extension tubes, suction tubes, etc.); catheters (e.g. urethral catheters, suction catheters, intravenous injection catheters, digestive tract catheters, etc.); bags (e.g. blood bags, infusion bags, liquid medicine bags, drain bags, etc.); circuit apparatus parts (e.g. blood component separators, hemodialysis circuits, peritoneal dialysis circuits, artificial cardiopulmonary bypass circuits, etc.); transfusion sets, blood transfusion sets, intravenous injection sets, cardiopulmonary bypasses, surgical gloves, packaging materials for pharmaceutical products, medical films, sanitary materials, respiration masks, nasal cannulas, connecting tubes, three-way stopcocks, connectors, winged needles, indwelling needles for dialysis, and the like.

In the vinyl chloride-based resin molded body, the rise in the haze value calculated by the following equation is preferably 6 or less, more preferably 5 or less, and particularly preferably 3 or less:

$$\text{Rise in haze value} = X - Y$$

wherein X is a haze value of a vinyl chloride-based resin molded body after irradiating with an electron beam at a dose of 50 kGy and then allowed to stand under a constant temperature and humidity condition of 25°C and 60% relative humidity for 10 days, and Y is a haze value of the vinyl chloride-based resin molded body before electron beam irradiation.

[Medical Materials]

The medical material of the present invention is obtained by sterilizing the vinyl chloride-based resin molded body by irradiation with radiation (electron beam). Even after sterilization by irradiation with radiation (electron beam), the medical material has very high transparency, low coloring, and excellent visibility of contents.

[Method for Improving Visibility of Contents]

The method for improving the visibility of contents according to the present invention is a method for improving visibility of contents of a material (for example, a medical material) sterilized by radiation (electron beam) irradiation wherein the material comprises a vinyl chloride-based resin molded body. The vinyl chloride-based resin molded body is characterized by a vinyl chloride-based resin molded body that is obtained by molding the vinyl chloride-based resin composition as a raw material comprising 5 to 100 parts by weight of a 4-cyclohexene-1,2-dicarboxylic acid diester and/or a 4,5-epoxycyclohexane-1,2-dicarboxylic acid diester with respect to 100 parts by weight of the vinyl chloride-based resin, into a desired shape.

EXAMPLES

Examples are shown below to more specifically explain the present invention. The present invention is not limited to these examples. Abbreviations of the compounds and measurements of the respective characteristics in examples and application examples are shown below.

[Measurement and Evaluation Method]

(1) Carbon Number of Saturated Aliphatic Alcohol and Ratio of Linear-Chain Saturated Aliphatic Alcohols

In the 4-cyclohexene-1,2-dicarboxylic acid diester or 4,5-epoxy-1,2-cyclohexanedicarboxylic acid diester used in Examples and Comparative Examples of the present invention,
the number of carbon atoms of the saturated aliphatic alcohol and the ratio of the linear-chain saturated aliphatic alcohols defining the residues represented by the general formula (1) or (2) are determined by measuring the composition of the raw material alcohol used in the production by gas chromatography (hereinafter abbreviated as GC). The results are used as the carbon number of the saturated aliphatic alcohol and the ratio of the linear-chain saturated aliphatic alcohols in these compounds. The method of measuring the raw material alcohol by GC is as follows.

<<GC Measurement Conditions>>

Apparatus: Gas chromatograph GC-17A (manufactured by Shimadzu Corporation)
Detector: FID
Column: Capillary column ZB-1 30 m
Column temperature: raised from 60°C to 290°C, temperature rise rate: 13°C/min
Carrier gas: Helium
Sample: 50% acetone solution
Injection volume: 1 μL
Quantitative determination: Quantitative determination was performed using n-propyl benzoate as an internal standard substance.

In selecting the internal standard substance, it has been confirmed in advance that n-propyl benzoate in the raw material alcohol is below the detection limit by GC.
In the esterification reaction and epoxidation reaction described above, there is no difference in reactivity or the like depending on the structure of the raw material alcohol within the scope of the present invention. It has been previously confirmed that there is no difference between the composition ratio in the raw material alcohol used and the composition ratio of the saturated aliphatic alcohol defining the residues in the general formula (1) or (2).

(2) Evaluation of Physical Properties of Stabilizer Compounds of Present Invention

The stabilizer compounds of the present invention obtained in the following Production Examples were analyzed by the following method.

Ester value: measured in accordance with JIS K-0070 (1992).
Acid value: measured in accordance with JIS K-0070 (1992).
Iodine value: measured in accordance with JIS K-0070 (1992).
Oxirane oxygen: measured in accordance with The JOCS Standard Methods for the Analysis of Fats, Oils and

Related Materials 2.3.7.1-2013 "Oxirane Oxygen Determination Method (Part 1)".
Color number: measured in accordance with JIS K-0071 (1998) to determine a Hazen unit color number.

(3) Preparation of Vinyl Chloride Sheet

To 100 parts by weight of a vinyl chloride resin (straight, degree of polymerization 1050, trade name "Zest 1000Z", manufactured by SHINDAI-ICHI VINYL Corporation) were respectively added 0.3 parts by weight of calcium stearate (manufactured by Nacalai Tesque Co., Ltd.) and 0.2 parts by weight of zinc stearate (manufactured by Nacalai Tesque Co., Ltd.) as a stabilizer other than the stabilizer of the present invention. The mixture was stirred and mixed with a mortar mixer, and then a predetermined amount of a stabilizer according to the present invention shown in Tables 1 to 4 and, if necessary, a predetermined amount of a plasticizer shown in Tables 1 to 4 were added until the mixture became uniform by handling and mixing, thereby to obtain a vinyl chloride-based resin composition of the present invention. This vinyl chloride-based resin composition was melt-kneaded at 160 to 166°C for 4 minutes using a 5 × 12 inches twin roll to prepare a roll sheet. Subsequently, press molding of the roll sheet was performed at 162 to 168°C for 10 minutes to prepare a press sheet having a thickness of about 1 mm.

(4) Electron Beam Irradiation Test

The press sheet obtained in the preceding section was cut into a size of 50 mm × 50 mm to obtain a test sample. Next, using the obtained sample, the sample was irradiated with an electron beam from a 10 MeV electron accelerator using an electron beam irradiation system of Nuclear Fuel Industries, Ltd. The doses of the irradiated electron beam were all adjusted to be 50 kGy.

(5) Haze Value

Using a haze meter (Haze-GARD2) manufactured by Toyo Seiki Seisaku-sho, Ltd., a haze value of each of a test piece before electron beam irradiation and a test piece that was allowed to stand for 10 days under constant temperature and humidity conditions of 25°C and 60% relative humidity after electron beam irradiation (a test piece on day 10 after electron beam irradiation) was measured in accordance with ASTM D1003. The rise in the haze value by electron beam irradiation was defined as a value obtained by subtracting the value of the test piece before irradiation from the value of the test piece on the 10th day after electron beam irradiation. It should be noted that as the numerical value of the obtained haze value becomes smaller, the transparency becomes more excellent or the transparency becomes less decrease.

(6) Yellow Index (YI Value)

Using a spectral colorimeter (CM-5) manufactured by Konica Minolta, Inc., a yellow index of each of a test piece before electron beam irradiation and a test piece that was allowed to stand for 10 days under constant temperature and humidity conditions of 25°C and 60% relative humidity after electron beam irradiation (a test piece on day 10 after electron beam irradiation) was measured in accordance with ASTM D1925. The rise in the yellow index due to electron beam irradiation was defined as a value obtained by subtracting the value of the test piece before irradiation from the value of the test piece on the 10th day after electron beam irradiation. In addition, it is shown that the smaller the numerical value of the obtained yellow index, the less the coloring.

<First Experimental Example>

[Production Example 1]

A 1 L four-necked flask equipped with a thermometer, a decanter, a stirring blade, and a reflux condenser was charged with 182.6 g of 4-cyclohexene-1,2-dicarboxylic anhydride (1.2 moles, RICACID TH, manufactured by New Japan Chemical Co., Ltd.), 374g (2.9 moles) of 2-ethylhexyl alcohol, and 0.24 g of tetraisopropyl titanate as an esterification catalyst, and then the esterification reaction was carried out at a reaction temperature of 200°C. The reaction was carried out until the acid value of the reaction solution became 0.5 mg KOH/g while removing the produced water out of the system by refluxing the alcohol under reduced pressure. After completion of the reaction, the unreacted alcohol was distilled out of the system under reduced pressure, and the system was neutralized, washed, and dehydrated according to a conventional method, thereby to obtain 369 g of a 4-cyclohexene-1,2-dicarboxylic acid diester (hereinafter referred to as "compound 1") as a stabilizer compound of the present invention.

The compound 1 thus obtained had an ester value of 283 mg KOH/g, an acid value of 0.01 mg KOH/g, and a

color number of 10.

[Production Example 2]

A 4-cyclohexene-1,2-dicarboxylic acid diester (449 g) (hereinafter, referred to as "compound 2") as a stabilizer compound of the present invention was obtained in the same manner as in Production Example 1, except that 416 g (2.9 moles) of an aliphatic saturated alcohol (manufactured by Shell Chemicals, product name: Linevol 9) comprising 88.5 mol% of a linear-chain saturated aliphatic alcohol having 9 carbon atoms and 11.1 mol% of a branched-chain saturated aliphatic alcohol having 9 carbon atoms was added in place of 374 g (2.9 moles) of 2-ethylhexyl alcohol.

The compound 2 thus obtained had an ester value of 260 mg KOH/g, an acid value of 0.04 mg KOH/g, and a color number of 10.

[Production Example 3]

A 4-cyclohexene-1,2-dicarboxylic acid diester (483 g) (hereinafter, referred to as "compound 3") as a stabilizer compound of the present invention was obtained in the same manner as in Production Example 1, except that 464 g (2.9 moles) of a mixed saturated aliphatic alcohol (manufactured by Shell Chemicals Co., Ltd., product name: Neodol 911) with 9/10/11 carbon atoms having a ratio (molar ratio) of 19/43/38 and an overall linear-chain ratio of 84% by weight was added in place of 374 g (2.9 moles) of 2-ethylhexyl alcohol.

The compound 3 thus obtained had an ester value of 247 mg KOH/g, an acid value of 0.02 mg KOH/g, and a color number of 5.

[Example 1]

According to the method of "(3) Preparation of Vinyl Chloride Sheet", a vinyl chloride-based resin composition was obtained by using 5 parts by weight of compound 1 obtained in Production Example 1 as a stabilizer, and 50 parts by weight of a commercially available tri-2-ethylhexyl trimellitate (TOTM) as a plasticizer, and then a vinyl chloride sheet was prepared from the obtained vinyl chloride-based resin composition.

Next, a test piece cut out from the obtained vinyl chloride sheet was irradiated with an electron beam under the conditions described in the above "(4) Electron Beam Irradiation Test". For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Example 2]

A vinyl chloride sheet was prepared in the same manner as in Example 1 except that 10 parts by weight of compound 1 was used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Example 3]

A vinyl chloride sheet was prepared in the same manner as in Example 1 except that 20 parts by weight of compound 1 was used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Example 4]

A vinyl chloride sheet was prepared in the same manner as in Example 2 except that compound 2 was used in place of compound 1, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Example 5]

A vinyl chloride sheet was prepared in the same manner as in Example 2 except that compound 3 was used in place of compound 1, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Example 6]

A vinyl chloride sheet was prepared in the same manner as in Example 2 except that diisononyl 1,2-cyclohexanedicarboxylate (manufactured by BASF, Hexamoll DINCH) was used in place of TOTM, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Example 7]

A vinyl chloride sheet was prepared in the same manner as in Example 2 except that di-2-ethylhexyl phthalate (manufactured by New Japan Chemical Co., Ltd., SANSO CIZER DOP) was used in place of TOTM, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Example 8]

A vinyl chloride sheet was prepared in the same manner as in Example 1 except that 50 parts by weight of compound 1 was used and TOTM was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Example 9]

A vinyl chloride sheet was prepared in the same manner as in Example 4 except that 50 parts by weight of compound 2 was used and TOTM was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Example 10]

A vinyl chloride sheet was prepared in the same manner as in Example 5 except that 50 parts by weight of compound 3 was used and TOTM was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Comparative Example 1]

A vinyl chloride sheet was prepared in the same manner as in Example 1 except that compound 1 was not used, and an electron beam was irradiated to a test piece cut out from the obtained vinyl chloride sheet. For the test piece before the electron beam irradiation and the test piece on the 10th day after the electron beam irradiation, the

haze and yellow index (YI value) of each test piece were measured. Table 1 shows the numerical values of the test pieces on the 10th day after the irradiation and the numerical values of the rise values.

[Comparative Example 2]

A vinyl chloride sheet was prepared in the same manner as in Example 6 except that compound 1 was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Comparative Example 3]

A vinyl chloride sheet was prepared in the same manner as in Example 7 except that compound 1 was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 1 shows the numerical values of the test pieces on the 10th day after the irradiation and the numerical values of the rise values.

[Table 1]

| | | Example | | | | | | | | | | Comparative Example | | |
|---|---|---|---|---|---|---|---|---|---|---|---|---|---|---|
| | | 1 | 2 | 3 | 4 | 5 | 6 | 7 | 8 | 9 | 10 | 1 | 2 | 3 |
| Stabilizer | Kind | Compound 1 | Compound 1 | Compound 1 | Compound 2 | Compound 3 | Compound 1 | Compound 1 | Compound 1 | Compound 2 | Compound 3 | - | - | - |
| | Blending amount (parts by weight) | 5 | 10 | 20 | 10 | 10 | 10 | 10 | 50 | 50 | 50 | - | - | - |
| Plasticizer | Kind | TOTM | TOTM | TOTM | TOTM | TOTM | DINCH | DOP | - | - | - | TOTM | DINCH | DOP |
| | Blending amount (parts by weight) | 50 | 50 | 50 | 50 | 50 | 50 | 50 | - | - | - | 50 | 50 | 50 |
| Haze value on day 10 after electron beam irradiation | | 12.8 | 6.7 | 6.0 | 6.3 | 4.3 | 5.5 | 7. 3 | 5.2 | 5.1 | 6.9 | 17.1 | 9.6 | 11.1 |
| Rise in haze value by electron beam irradiation | | 5. 9 | 2.9 | 1.1 | 2.5 | 0. 9 | 0.2 | 1.4 | 1.8 | -0.1 | -0.2 | 13.7 | 4.7 | 7. 5 |
| YI value on day 10 after electron beam irradiation | | 52.8 | 43.4 | 32.3 | 48.7 | 38.4 | 35.1 | 41.5 | 26.5 | 23.7 | 31.8 | 77.0 | 59.9 | 67.4 |
| Rise in YI value by electron beam irradiation | | 35.6 | 16.0 | 12.4 | 17.1 | 14.1 | 13.3 | 9.8 | 15.4 | 12.4 | 21.2 | 58.7 | 49.4 | 51.7 |

From the comparison between the results of Examples 1 to 7 and the results of Comparative Examples 1 to 3 in Table 1, it was found that by adding a 4-cyclohexene-1,2-dicarboxylic acid diester as a stabilizer of the present invention to a vinyl chloride sheet, the haze value and the yellow index caused by the irradiation with an electron beam were suppressed from rising, and as a result, sufficient transparency and hue were maintained even after 10 days from the sterilization by electron beam irradiation so that the contents can be visually recognized. In addition, it was found that the effect was more remarkably exhibited in a vinyl chloride sheet imparted with plasticity by increasing the amount of the stabilizer of the present invention without using other plasticizers (results of Examples 8 to 10). It is clear from the above results that the stabilizer of the present invention is very effective in improving the visibility of the contents of medical materials after electron beam irradiation sterilization.

<Second Experimental Example>

[Production Example 4]

[Esterification Reaction]

A 1 L four-necked flask equipped with a thermometer, a decanter, a stirring blade, and a reflux condenser was charged with 182.6 g of 4-cyclohexene-1,2-dicarboxylic anhydride (1.2 moles, RICACID TH, manufactured by New Japan Chemical Co., Ltd.), 374g (2.9 moles) of 2-ethylhexyl alcohol, and 0.24 g of tetraisopropyl titanate as an esterification catalyst, and then the esterification reaction was carried out at a reaction temperature of 200°C. The reaction was carried out until the acid value of the reaction solution became 0.5 mg KOH/g while removing the produced water out of the system by refluxing the alcohol under reduced pressure. After completion of the reaction, the unreacted alcohol was distilled out of the system under reduced pressure, and the system was neutralized, washed, and dehydrated according to a conventional method, thereby to obtain 404 g of a 4-cyclohexene-1,2-dicarboxylic acid diester as an intermediate raw material.

[Epoxidation Reaction]

Then, a 1 L four-necked flask equipped with a thermometer, a stirring blade, and a condenser was charged with 394 g (1.0 mole) of the 4-cyclohexene-1,2-dicarboxylic acid diester obtained by the above esterification reaction, and the temperature was raised to 60 to 70°C. After raising the temperature, 76.6 g (1.35 moles) of 60% hydrogen peroxide water, 18.3 g (0.30 moles) of 76% formic acid, and 1.47 g (0.01 moles) of 75% phosphoric acid were slowly added dropwise over a period of 2 hours and 15 minutes. After completion of the dropwise addition, the above temperature was maintained for further 4 hours, and the reaction was completed by aging. After completion of the reaction, the aqueous phase was removed to the outside of the system, followed by washing with water and dehydration in a conventional manner to obtain 370 g of a 4,5-epoxycyclohexane dicarboxylic acid diester (hereinafter referred to as "compound 4") as an objective stabilizer of the present invention.

The compound 4 thus obtained had an ester value of 273 mg KOH/g, an acid value of 0.04 mg KOH/g, an iodine value of 2.6 g $I_2$/100 g, an oxirane oxygen content of 3.7%, and a color number of 10.

[Production Example 5]

A 4,5-epoxycyclohexane dicarboxylic acid diester (397 g) (hereinafter, referred to as "compound 5") as a stabilizer compound of the present invention was obtained in the same manner as in Production Example 4, except that 416 g (2.9 moles) of an aliphatic saturated alcohol (manufactured by Shell Chemicals, product name: Linevol 9) comprising 88.5 mol% of a linear-chain saturated aliphatic alcohol having 9 carbon atoms and 11.1 mol% of a branched-chain saturated aliphatic alcohol having 9 carbon atoms was added in place of 378 g (2.9 moles) of 2-ethylhexyl alcohol.

The compound 5 thus obtained had an ester value of 256 mg KOH/g, an acid value of 0.06 mg KOH/g, an iodine value of 1.7 g $I_2$/100 g, an oxirane oxygen content of 3.5%, and a color number of 10.

[Production Example 6]

A 4,5-epoxycyclohexane dicarboxylic acid diester (404 g) (hereinafter, referred to as "compound 6") as a stabilizer compound of the present invention was obtained in the same manner as in Production Example 4, except that 464 g (2.9 moles) of a mixed saturated aliphatic alcohol (manufactured by Shell Chemicals Co., Ltd., product name: Neodol 911) with 9/10/11 carbon atoms having a ratio (molar ratio) of 19/43/38 and an overall linear-chain ratio of 84% by weight was added in place of 378 g (2.9 moles) of 2-ethylhexyl alcohol.

The compound 6 thus obtained had an ester value of 242 mg KOH/g, an acid value of 0.04 mg KOH/g, an

iodine value of 1.7 g $I_2$/100 g, an oxirane oxygen content of 3.1%, and a color number of 10.

[Example 11]

According to the method of "(3) Preparation of Vinyl Chloride Sheet", a vinyl chloride-based resin composition was obtained by using 5 parts by weight of compound 4 obtained in Production Example 4 as a stabilizer, and 50 parts by weight of a commercially available tri-2-ethylhexyl trimellitate (TOTM) as a plasticizer, and then a vinyl chloride sheet was prepared from the obtained vinyl chloride-based resin composition.

Next, a test piece cut out from the obtained vinyl chloride sheet was irradiated with an electron beam under the conditions described in the above "(4) Electron Beam Irradiation Test". For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Example 12]

A vinyl chloride sheet was prepared in the same manner as in Example 11 except that 10 parts by weight of compound 4 was used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Example 13]

A vinyl chloride sheet was prepared in the same manner as in Example 11 except that 20 parts by weight of compound 4 was used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Example 14]

A vinyl chloride sheet was prepared in the same manner as in Example 12 except that compound 5 was used in place of compound 4, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Example 15]

A vinyl chloride sheet was prepared in the same manner as in Example 12 except that compound 6 was used in place of compound 4, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Example 16]

A vinyl chloride sheet was prepared in the same manner as in Example 12 except that diisononyl 1,2-cyclohexanedicarboxylate (manufactured by BASF, Hexamoll DINCH) was used in place of TOTM, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Example 17]

A vinyl chloride sheet was prepared in the same manner as in Example 12 except that di-2-ethylhexyl phthalate

(manufactured by New Japan Chemical Co., Ltd., SANSO CIZER DOP) was used in place of TOTM, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Example 18]

A vinyl chloride sheet was prepared in the same manner as in Example 11 except that 50 parts by weight of compound 4 was used and TOTM was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Example 19]

A vinyl chloride sheet was prepared in the same manner as in Example 14 except that 50 parts by weight of compound 5 was used and TOTM was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Example 20]

A vinyl chloride sheet was prepared in the same manner as in Example 15 except that 50 parts by weight of compound 6 was used and TOTM was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Comparative Example 4]

A vinyl chloride sheet was prepared in the same manner as in Example 11 except that compound 4 was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Comparative Example 5]

A vinyl chloride sheet was prepared in the same manner as in Example 16 except that compound 4 was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Comparative Example 6]

A vinyl chloride sheet was prepared in the same manner as in Example 17 except that compound 4 was not used, and an electron beam was irradiated to a test piece cut out from the obtained vinyl chloride sheet. For the test piece before the electron beam irradiation and the test piece on the 10th day after the electron beam irradiation, the haze and yellow index (YI value) of each test piece were measured. Table 2 shows the numerical values of the test pieces on the 10th day after the irradiation and the numerical values of the rise values.

[Table 2]

| | | EXAMPLE | | | | | | | | | | Comparative Example | | |
|---|---|---|---|---|---|---|---|---|---|---|---|---|---|---|
| | | 11 | 12 | 13 | 14 | 15 | 16 | 17 | 18 | 19 | 20 | 4 | 5 | 6 |
| Stabilizer | Kind | Compound 4 | Compound 4 | Compound 4 | Compound 5 | Compound 6 | Compound 4 | Compound 4 | Compound 4 | Compound 5 | Compound 6 | - | - | - |
| | Blending amount (parts by weight) | 5 | 10 | 20 | 10 | 10 | 10 | 10 | 50 | 50 | 50 | - | - | - |
| Plasticizer | Kind | TOTM | TOTM | TOTM | TOTM | TOTM | DINCH | DOP | - | - | - | TOTM | DINCH | DOP |
| | Blending amount (parts by weight) | 50 | 50 | 50 | 50 | 50 | 50 | 50 | - | - | - | 50 | 50 | 50 |
| Haze value on day 10 after electron beam irradiation | | 2.5 | 2.1 | 2.5 | 1. 9 | 2.1 | 3.4 | 2.9 | 2.1 | 1.8 | 2.4 | 17.1 | 9.6 | 11.1 |
| Rise in haze value by electron beam irradiation | | 0.7 | 0.2 | 0.3 | 0.1 | 0.5 | 1.4 | 0.6 | 0.5 | -0.2 | 0. 3 | 13.7 | 4. 7 | 7. 5 |
| YI value on day 10 after electron beam irradiation | | 41.1 | 34.1 | 29.0 | 31.9 | 36.7 | 22.4 | 26.1 | 27.3 | 27.7 | 31.5 | 77.0 | 59.9 | 67.4 |
| Rise in YI value by electron beam irradiation | | 12.7 | 6.7 | 4.5 | 1.5 | 3.1 | 8.0 | 6.4 | 6.3 | -8.6 | -9.9 | 58.7 | 49.4 | 51.7 |

From the comparison between the results of Examples 11 to 17 and the results of Comparative Examples 4 to 6 in Table 2, it was found that by adding a 4,5-epoxycyclohexane-1,2-dicarboxylic acid diester as a stabilizer of the present invention to a vinyl chloride sheet, the haze value and the yellow index caused by the irradiation with an electron beam were suppressed from rising, and as a result, sufficient transparency and hue were maintained even after 10 days from the sterilization by electron beam irradiation so that the contents can be visually recognized. In addition, it was found that the effect was more remarkably exhibited in a vinyl chloride sheet imparted with plasticity by increasing the amount of the stabilizer of the present invention without using other plasticizers (results of Examples 18 to 20). It is clear from the above results that the stabilizer of the present invention is very effective in improving the visibility of the contents of medical materials after electron beam irradiation sterilization.

<Third Experimental Example>

[Production Example 7]

A 1 L four-necked flask equipped with a thermometer, a decanter, a stirring blade, and a reflux condenser was charged with 182.6 g of 4-cyclohexene-1,2-dicarboxylic anhydride (1.2 moles, RICACID TH, manufactured by New Japan Chemical Co., Ltd.), 374g (2.9 moles) of 2-ethylhexyl alcohol, and 0.24 g of tetraisopropyl titanate as an esterification catalyst, and then the esterification reaction was carried out at a reaction temperature of 200°C. The reaction was carried out until the acid value of the reaction solution became 0.5 mg KOH/g while removing the produced water out of the system by refluxing the alcohol under reduced pressure. After completion of the reaction, the unreacted alcohol was distilled out of the system under reduced pressure, and the system was neutralized, washed, and dehydrated according to a conventional method, thereby to obtain 369 g of a 4-cyclohexene-1,2-dicarboxylic acid diester (hereinafter referred to as "compound 1") as a stabilizer compound of the present invention.

The compound 1 thus obtained had an ester value of 283 mg KOH/g, an acid value of 0.01 mg KOH/g, and a color number of 10.

[Production Example 8]

A 4-cyclohexene-1,2-dicarboxylic acid diester (449 g) (hereinafter, referred to as "compound 2") as a stabilizer compound of the present invention was obtained in the same manner as in Production Example 7, except that 416 g (2.9 moles) of an aliphatic saturated alcohol (manufactured by Shell Chemicals, product name: Linevol 9) comprising 88.5 mol% of a linear-chain saturated aliphatic alcohol having 9 carbon atoms and 11.1 mol% of a branched-chain saturated aliphatic alcohol having 9 carbon atoms was added in place of 374 g (2.9 moles) of 2-ethylhexyl alcohol.

The compound 2 thus obtained had an ester value of 260 mg KOH/g, an acid value of 0.04 mg KOH/g, and a color number of 10.

[Production Example 9]

A 4-cyclohexene-1,2-dicarboxylic acid diester (483 g) (hereinafter, referred to as "compound 3") as a stabilizer compound of the present invention was obtained in the same manner as in Production Example 7, except that 464 g (2.9 moles) of a mixed saturated aliphatic alcohol (manufactured by Shell Chemicals Co., Ltd., product name: Neodol 911) with 9/10/11 carbon atoms having a ratio (molar ratio) of 19/43/38 and an overall linear-chain ratio of 84% by weight was added in place of 374 g (2.9 moles) of 2-ethylhexyl alcohol.

The compound 3 thus obtained had an ester value of 247 mg KOH/g, an acid value of 0.02 mg KOH/g, and a color number of 5.

[Example 21]

According to the method of "(3) Preparation of Vinyl Chloride Sheet", a vinyl chloride-based resin composition was obtained by using 50 parts by weight of compound 1 as a plasticizer and 0.2 parts by weight of a commercially available zinc acetylacetonate as a stabilizer. After that, a vinyl chloride sheet was prepared from the obtained vinyl chloride-based resin composition. Next, a test piece cut out from the obtained vinyl chloride sheet was irradiated with an electron beam under the conditions described in the above "(4) Electron Beam Irradiation Test". For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Example 22]

A vinyl chloride sheet was prepared in the same manner as in Example 21 except that 0.5 parts by weight of zinc acetylacetonate was used, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Example 23]

A vinyl chloride sheet was prepared in the same manner as in Example 21 except that 5 parts by weight of epoxidized soybean oil was further used, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Example 24]

A vinyl chloride sheet was prepared in the same manner as in Example 23 except that zinc dehydroacetate was used in place of zinc acetylacetonate, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Example 25]

A vinyl chloride sheet was prepared in the same manner as in Example 23 except that compound 2 was used in place of compound 1, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Example 26]

A vinyl chloride sheet was prepared in the same manner as in Example 23 except that compound 3 was used in place of compound 1, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Comparative Example 7]

A vinyl chloride sheet was prepared in the same manner as in Example 21 except that zinc acetylacetonate was not used, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Comparative Example 8]

A vinyl chloride sheet was prepared in the same manner as in Comparative Example 7 except that di-2-ethylhexyl phthalate (manufactured by New Japan Chemical Co., Ltd., SANSO CIZER DOP) was used in place of compound 1, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Comparative Example 9]

A vinyl chloride sheet was prepared in the same manner as in Example 21 except that di-2-ethylhexyl phthalate (manufactured by New Japan Chemical Co., Ltd., SANSO CIZER DOP) was used in place of compound 1, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Comparative Example 10]

A vinyl chloride sheet was prepared in the same manner as in Example 22 except that di-2-ethylhexyl phthalate (manufactured by New Japan Chemical Co., Ltd., SANSO CIZER DOP) was used in place of compound 1, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 3 shows the YI value of the test piece on day 10 after the electron beam irradiation.

[Table 3]

| | | Example 21 | Example 22 | Example 23 | Example 24 | Example 25 | Example 26 | Comparative Example 7 | Comparative Example 8 | Comparative Example 9 | Comparative Example 10 |
|---|---|---|---|---|---|---|---|---|---|---|---|
| Blending (parts) | Compound 1 | 50 | 50 | 50 | 50 | | | 50 | | | |
| | Compound 2 | | | | | 50 | | | | | |
| | Compound 3 | | | | | | 50 | | | | |
| | Di-2-ethylhexyl phthalate | | | | | | | | 50 | 50 | 50 |
| | Epoxidized soybean oil | | | 5 | 5 | 5 | 5 | | | | |
| | Zinc dehydroacetate | | | | 0.2 | | | | | | |
| | Zinc acetylacetonate | 0.2 | 0. 5 | 0.2 | | 0.2 | 0.2 | | | 0.2 | 0. 5 |
| YI value | YI value on day 10 after electron beam irradiation | 32.6 | 16.5 | 14.4 | 14.6 | 15.1 | 14.2 | 40.0 | 87.6 | 81.6 | 41.7 |
| | Rise in YI value by electron beam irradiation | 23.4 | 5. 4 | 5.3 | 3.8 | 4. 4 | 3. 7 | 28.9 | 71.8 | 71.1 | 28.8 |

From the comparison between the results of Examples 21 to 26 and the results of Comparative Examples 7 to 10 in Table 3, it is found that the rise in yellow index after electron beam irradiation is significantly suppressed by adding a 4-cyclohexene-1,2-dicarboxylic acid diester as a plasticizer of the present invention and a β-diketone compound as a stabilizer. It is further understood that the addition of an epoxy compound suppresses much more the rise in the yellow index after electron beam irradiation. As a result, it is found that a hue enough to identify the contents was maintained even after 10 days had passed after sterilization by electron beam irradiation. It is clear from the above results that the vinyl chloride-based resin composition of the present invention is very useful as a raw material of a medical material used after being sterilized by electron beam irradiation. Specifically, a vinyl chloride-based resin molded body obtained from the vinyl chloride-based resin composition of the present invention has a very good hue, so that such molded body has a very high visibility of the contents and can be used suitably as medical materials such as an infusion bag, an infusion tube, and a blood circuit.

<Fourth Experimental Example>

[Production Example 10]

[Esterification Reaction]

A 1 L four-necked flask equipped with a thermometer, a decanter, a stirring blade, and a reflux condenser was charged with 182.6 g of 4-cyclohexene-1,2-dicarboxylic anhydride (1.2 moles, RICACID TH, manufactured by New Japan Chemical Co., Ltd.), 374g (2.9 moles) of 2-ethylhexyl alcohol, and 0.24 g of tetraisopropyl titanate as an esterification catalyst, and then the esterification reaction was carried out at a reaction temperature of 200°C. The reaction was carried out until the acid value of the reaction solution became 0.5 mg KOH/g while removing the produced water out of the system by refluxing the alcohol under reduced pressure. After completion of the reaction, the unreacted alcohol was distilled out of the system under reduced pressure, and the system was neutralized, washed, and dehydrated according to a conventional method, thereby to obtain 404 g of a 4-cyclohexene-1,2-dicarboxylic acid diester as an intermediate raw material.

[Epoxidation Reaction]

Then, a 1 L four-necked flask equipped with a thermometer, a stirring blade, and a condenser was charged with 394 g (1.0 mole) of the 4-cyclohexene-1,2-dicarboxylic acid diester obtained by the above esterification reaction, and the temperature was raised to 60 to 70°C. After raising the temperature, 76.6 g (1.35 moles) of 60% hydrogen peroxide water, 18.3 g (0.30 moles) of 76% formic acid, and 1.47 g (0.01 moles) of 75% phosphoric acid were slowly added dropwise over a period of 2 hours and 15 minutes. After completion of the dropwise addition, the above temperature was maintained for further 4 hours, and the reaction was completed by aging. After completion of the reaction, the aqueous phase was removed to the outside of the system, followed by washing with water and dehydration in a conventional manner to obtain 370 g of a 4,5-epoxycyclohexane dicarboxylic acid diester (hereinafter referred to as "compound 4") as an objective stabilizer of the present invention.

The compound 4 thus obtained had an ester value of 273 mg KOH/g, an acid value of 0.04 mg KOH/g, an iodine value of 2.6 g $I_2$/100 g, an oxirane oxygen content of 3.7%, and a color number of 10.

[Production Example 11]

A 4,5-epoxycyclohexane dicarboxylic acid diester (397 g) (hereinafter, referred to as "compound 5") as a stabilizer compound of the present invention was obtained in the same manner as in Production Example 10, except that 416 g (2.9 moles) of an aliphatic saturated alcohol (manufactured by Shell Chemicals, product name: Linevol 9) comprising 88.5 mol% of a linear-chain saturated aliphatic alcohol having 9 carbon atoms and 11.1 mol% of a branched-chain saturated aliphatic alcohol having 9 carbon atoms was added in place of 378 g (2.9 moles) of 2-ethylhexyl alcohol.

The compound 5 thus obtained had an ester value of 256 mg KOH/g, an acid value of 0.06 mg KOH/g, an iodine value of 1.7 g $I_2$/100 g, an oxirane oxygen content of 3.5%, and a color number of 10.

[Production Example 12]

A 4,5-epoxycyclohexane dicarboxylic acid diester (404 g) (hereinafter, referred to as "compound 6") as a stabilizer compound of the present invention was obtained in the same manner as in Production Example 10, except that 464 g (2.9 moles) of a mixed saturated aliphatic alcohol (manufactured by Shell Chemicals Co., Ltd., product name: Neodol 911) with 9/10/11 carbon atoms having a ratio (molar ratio) of 19/43/38 and an overall linear-chain ratio of 84%

by weight was added in place of 378 g (2.9 moles) of 2-ethylhexyl alcohol.

The compound 6 thus obtained had an ester value of 242 mg KOH/g, an acid value of 0.04 mg KOH/g, an iodine value of 1.7 g $I_2$/100 g, an oxirane oxygen content of 3.1%, and a color number of 10.

[Example 27]

According to the method of "(3) Preparation of Vinyl Chloride Sheet", a vinyl chloride-based resin composition was obtained by using 50 parts by weight of compound 4 as a plasticizer and 0.5 parts by weight of a commercially available zinc acetylacetonate as a stabilizer. After that, a vinyl chloride sheet was prepared from the obtained vinyl chloride-based resin composition. Next, a test piece cut out from the obtained vinyl chloride sheet was irradiated with an electron beam under the conditions described in the above "(4) Electron Beam Irradiation Test". For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Example 28]

A vinyl chloride sheet was prepared in the same manner as in Example 27 except that 0.2 parts by weight of zinc acetylacetonate was used and 5 parts by weight of epoxidized soybean oil was further used, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Example 29]

A vinyl chloride sheet was prepared in the same manner as in Example 27 except that 0.2 parts by weight of zinc dehydroacetate was used in place of 0.5 parts by weight of zinc acetylacetonate, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Example 30]

A vinyl chloride sheet was prepared in the same manner as in Example 28 except that zinc dehydroacetate was used in place of zinc acetylacetonate, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Example 31]

A vinyl chloride sheet was prepared in the same manner as in Example 27 except that compound 5 was used in place of compound 4 and 0.2 parts by weight of zinc acetylacetonate was used, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Example 32]

A vinyl chloride sheet was prepared in the same manner as in Example 31 except that 5 parts by weight of epoxidized soybean oil was further used, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Example 33]

A vinyl chloride sheet was prepared in the same manner as in Example 32 except that compound 6 was used in place of compound 5, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Comparative Example 11]

A vinyl chloride sheet was prepared in the same manner as in Example 27 except that zinc acetylacetonate was not used, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Comparative Example 12]

A vinyl chloride sheet was prepared in the same manner as in Comparative Example 11 except that di-2-ethylhexyl phthalate (manufactured by New Japan Chemical Co., Ltd., SANSO CIZER DOP) was used in place of compound 4, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Comparative Example 13]

A vinyl chloride sheet was prepared in the same manner as in Comparative Example 12 except that 0.2 parts by weight of zinc acetylacetonate was further used, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Comparative Example 14]

A vinyl chloride sheet was prepared in the same manner as in Comparative Example 13 except that 0.5 parts by weight of zinc acetylacetonate was used, and an electron beam was irradiated to a test piece cut out from the obtained vinyl chloride sheet. For the test piece before the electron beam irradiation, the test piece immediately after the electron beam irradiation, and the test piece on day 10 after the electron beam irradiation, the yellow index (YI value) of each test piece was measured. Table 4 shows the YI value of the test piece on day 10 after the electron beam irradiation.

[Table 4]

| | | Example 27 | Example 28 | Example 29 | Example 30 | Example 31 | Example 32 | Example 33 | Comparative Example 11 | Comparative Example 12 | Comparative Example 13 | Comparative Example 14 |
|---|---|---|---|---|---|---|---|---|---|---|---|---|
| Blending (parts) | Compound 4 | 50 | 50 | 50 | 50 | | | | 50 | | | |
| | Compound 5 | | | | | 50 | 50 | | | | | |
| | Compound 6 | | | | | | | 50 | | | | |
| | Di-2-ethylhexyl phthalate | | | | | | | | | 50 | 50 | 50 |
| | Epoxidized soybean oil | | 5 | | 5 | | 5 | 5 | | | | |
| | Zinc dehydroacetate | | | 0.2 | 0.2 | | | | | | | |
| | Zinc acetylacetonate | 0. 5 | 0.2 | | | 0.2 | 0.2 | 0.2 | | | 0.2 | 0. 5 |
| YI value | YI value on day 10 after electron beam irradiation | 20.6 | 17.6 | 19.4 | 19.1 | 22.1 | 17.6 | 21.7 | 29.2 | 87.6 | 81.6 | 41.7 |
| | Rise in YI value by electron beam irradiation | 5.5 | 6.0 | 6.8 | 6.4 | 6.9 | 5.0 | 5. 4 | 8.3 | 71.8 | 71.1 | 28.8 |

From the comparison between the results of Examples 27 to 33 and the results of Comparative Examples 11 to 14 in Table 4, it is found that the rise in yellow index after electron beam irradiation is significantly suppressed by adding a 4,5-epoxycyclohexane dicarboxylic acid diester as a plasticizer of the present invention and a β-diketone compound as a stabilizer. It is further understood that the addition of an epoxy compound suppresses much more the rise in the yellow index after electron beam irradiation. As a result, it is found that a hue enough to identify the contents was maintained even after 10 days had passed after sterilization by electron beam irradiation. It is clear from the above results that the vinyl chloride-based resin composition of the present invention is very useful as a raw material of a medical material used after being sterilized by electron beam irradiation. Specifically, a vinyl chloride-based resin molded body obtained from the vinyl chloride-based resin composition of the present invention has a very good hue, so that such molded body has a very high visibility of the contents and can be used suitably as medical materials such as an infusion bag, an infusion tube, and a blood circuit.

INDUSTRIAL APPLICABILITY

By using a stabilizer of the present invention containing a 4-cyclohexene-1,2-dicarboxylic acid diester and/or a 4,5-epoxycyclohexane-1,2-dicarboxylic acid diester, and a method to improve the visibility of the contents using the stabilizer, or by using a vinyl chloride-based resin composition containing (a) a 4-cyclohexene-1,2-dicarboxylic acid diester and/or a 4,5-epoxycyclohexane-1,2-dicarboxylic acid diester and (b) a β-diketone compound or its metal salt, loss of transparency and coloring that occur over time during and after electron beam irradiation can be reduced, so that the visibility of the contents of a medical material sterilized by electron beam irradiation is greatly improved. As a result, the problem of reduction in the visibility of the contents of the medical material after electron beam irradiation sterilization, which has been a problem so far, is solved, and medical accidents and the like can be reduced. Therefore, the medical material of the present invention can be used suitably as materials for various medical devices for medical tubes such as dialysis circuit tubes and catheters, medical bags such as blood bags and infusion bags, blood circuits such as connecting members and various valves, and syringes.

**Claims**

1. A method for improving visibility of contents of a material containing a vinyl chloride-based resin molded body that has been subjected to electron beam irradiation sterilization, wherein the vinyl chloride-based resin molded body contains 5 to 100 parts by weight of a 4-cyclohexene-1,2-dicarboxylic acid diester and/or a 4,5-epoxycyclohexane-1,2-dicarboxylic acid diester with respect to 100 parts by weight of a vinyl chloride-based resin.

2. The method for improving the visibility of the contents according to claim 1, wherein the 4-cyclohexene-1,2-dicarboxylic acid diester is a compound represented by the following general formula (1):

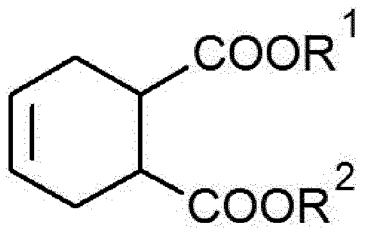

(1)

wherein $R^1$ and $R^2$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms.

3. The method for improving the visibility of the contents according to claim 1 or 2, wherein the 4,5-epoxycyclohexane-1,2-dicarboxylic acid diester is a compound represented by the following general formula (2):

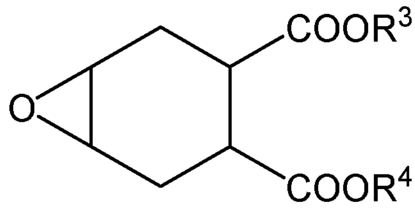

(2)

wherein $R^3$ and $R^4$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms.

**4.** A vinyl chloride-based resin composition for a medical material having improved visibility of contents after electron beam irradiation sterilization, the vinyl chloride-based resin composition comprising 100 parts by weight of a vinyl chloride-based resin and 5 to 100 parts by weight of a 4-cyclohexene-1,2-dicarboxylic acid diester and/or a 4,5-epoxycyclohexane-1,2-dicarboxylic acid diester.

**5.** A stabilizer for improving visibility of contents of a material containing a vinyl chloride-based resin molded body after electron beam irradiation sterilization, the stabilizer comprising a 4-cyclohexene-1,2-dicarboxylic acid diester represented by the following general formula (1) :

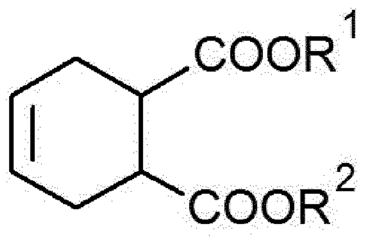

(1)

wherein $R^1$ and $R^2$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms
and/or a 4,5-epoxycyclohexane-1,2-dicarboxylic acid diester represented by the following general formula (2):

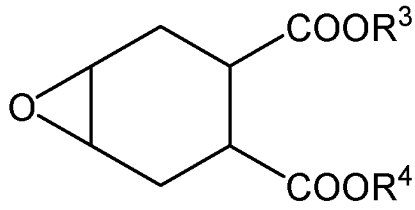

(2)

wherein $R^3$ and $R^4$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms.

**6.** A vinyl chloride-based resin molded body comprising the stabilizer according to claim 5.

**7.** The vinyl chloride-based resin molded body according to claim 6, wherein the content of the stabilizer is 5 to 100 parts by weight with respect to 100 parts by weight of a vinyl chloride-based resin.

**8.** A medical material sterilized by electron beam irradiation, comprising the vinyl chloride-based resin molded body according to claim 6 or 7.

**9.** A vinyl chloride-based resin composition that is a raw material for a medical material sterilized by irradiation with radiation, the vinyl chloride-based resin composition comprising, with respect to 100 parts by weight of a vinyl chloride-based resin:

(a) 5 to 100 parts by weight of a 4-cyclohexene-1,2-dicarboxylic acid diester represented by a following general formula (1):

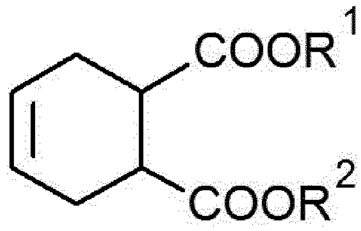

(1)

wherein $R^1$ and $R^2$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms
and/or a 4,5-epoxycyclohexane-1,2-dicarboxylic acid diester represented by the following general formula (2):

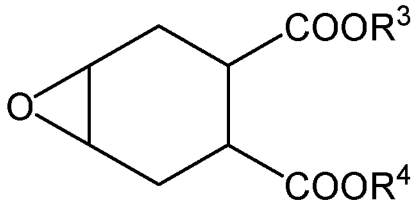

(2)

wherein $R^3$ and $R^4$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms; and
(b) 0.01 to 1.0 part by weight of at least one selected from a group consisting of a β-diketone compound and a metal salt thereof.

**10.** The vinyl chloride-based resin composition according to claim 9, wherein the β-diketone compound and the metal salt thereof (b) are at least one compound selected from a group consisting of zinc dehydroacetate, calcium acetylacetonate, zinc acetylacetonate, and magnesium acetylacetonate.

**11.** The vinyl chloride-based resin composition according to claim 9 or 10, further comprising 0.1 to 30 parts by weight of (c) an epoxy compound other than the 4,5-epoxycyclohexane-1,2-dicarboxylic acid diester represented by the general formula (2).

**12.** The vinyl chloride-based resin composition according to claim 11, wherein the epoxy compound (c) is an epoxidized soybean oil.

**13.** The vinyl chloride-based resin composition according to any one of claims 9 to 12, wherein the radiation is an electron beam.

**14.** A vinyl chloride-based resin molded body for a medical material, the vinyl chloride-based resin molded body being obtained from the vinyl chloride-based resin composition according to any one of claims 9 to 13.

**15.** A sterilization processing method by radiation irradiation of a vinyl chloride-based resin molded body for a medical material, the vinyl chloride-based resin molded body being obtained from a vinyl chloride-based resin composition, wherein the vinyl chloride-based resin composition contains, with respect to 100 parts by weight of a vinyl chloride-based resin:

(a) 5 to 100 parts by weight of a 4-cyclohexene-1,2-dicarboxylic acid diester represented by the following general formula (1):

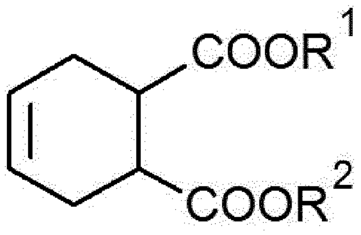

(1)

wherein $R^1$ and $R^2$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms
and/or a 4,5-epoxycyclohexane-1,2-dicarboxylic acid diester represented by the following general formula (2):

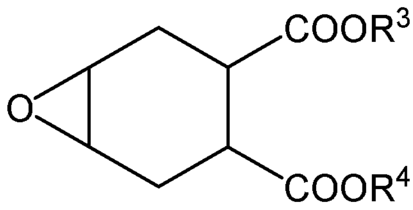

(2)

wherein $R^3$ and $R^4$ are the same or different and each represents a residue obtained by removing a hydroxyl group from a saturated aliphatic alcohol, and the saturated aliphatic alcohol is a linear or branched-chain saturated aliphatic alcohol having 7 to 13 carbon atoms; and
(b) 0.01 to 1.0 part by weight of at least one selected from the group consisting of a β-diketone compound and a metal salt thereof.

**16.** The sterilization processing method by radiation irradiation of the vinyl chloride-based resin molded body for the medical material according to claim 15, wherein the vinyl chloride-based resin composition further contains 0.1 to 30 parts by weight of (c) an epoxy compound other than the 4,5-epoxycyclohexane-1,2-dicarboxylic acid diester represented by the general formula (2).

**17.** The sterilization processing method by radiation irradiation of the vinyl chloride-based resin molded body for the medical material according to claim 15 or 16, wherein the radiation is an electron beam.

## INTERNATIONAL SEARCH REPORT

International application No.
PCT/JP2018/042838

A. CLASSIFICATION OF SUBJECT MATTER
Int.Cl. C08L27/06(2006.01)i, C08K5/12(2006.01)i

According to International Patent Classification (IPC) or to both national classification and IPC

B. FIELDS SEARCHED

Minimum documentation searched (classification system followed by classification symbols)
Int.Cl. C08L27/06, C08K5/12

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2015/147300 A1 (NEW JAPAN CHEMICAL CO., LTD.) 01 October 2015, claims, paragraphs [0040]-[0043], [0168], [0174], examples & JP 2016-6145 A & JP 2016-141712 A & JP 2016-141729 A & JP 2016-141787 A & JP 2016-150966 A & JP 2016-155938 A & JP 2015-193817 A & JP 2016-74876 A & JP 2016-89155 A & US 2017/0015810 A1; claims, paragraphs [0064]-[0067], [0244], [0250], examples & EP 3124540 A1 & CN 106164164 A & KR 10-2016-0139001 A & TW 201542639 A | 1-8<br>9-17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 February 2019 (15.02.2019) | 26 February 2019 (26.02.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/042838

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2016/190354 A1 (NEW JAPAN CHEMICAL CO., LTD.) 01 December 2016, claims, paragraphs [0037], [0194], [0196], examples & JP 2017-36225 A & JP 2017-148199 A & JP 2016-222895 A & JP 2017-14480 A & JP 2017-25296 A & US 2018/0127390 A1; claims, paragraphs [0062]-[0066], [0226], [0228], examples & EP 3305772 A1 & CN 107614492 A & KR 10-2018-0012318 A | 1-8<br>9-17 |
| Y<br>A | JP 2015-187223 A (SAKAI CHEMICAL INDUSTRY CO., LTD.) 29 October 2015, claims, paragraphs [0006], [0037]-[0039], examples (Family: none) | 9-17<br>1-8 |
| Y<br>A | JP 2005-097394 A (ASAHI DENKA KOGYO KK.) 14 April 2005, claims, paragraphs [0009], [0023], [0031], examples (Family: none) | 9-17<br>1-8 |
| Y<br>A | WO 2012/014583 A1 (ADEKA CORPORATION) 02 February 2012, claims, paragraphs [0018], [0039], examples & US 2013/0131227 A1; claims, paragraphs [0021], [0042], examples & EP 2599830 A1 & CN 103025824 A & KR 10-2013-0141453 A & KR 10-2018-0113646 A | 9-17<br>1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S59164066 A
- JP 2006239026 A
- JP H824329 A
- JP 2008169332 A
- JP 2015028116 A
- JP H2263853 A
- JP 2000273259 A
- WO 2015129534 A
- JP H2222436 A
- JP 2013100549 A
- JP 2012057099 A

### Non-patent literature cited in the description

- Oxirane Oxygen Determination Method (Part 1). *Analysis of Fats, Oils and Related Materials 2.3.7.1-2013*
- Oxirane Oxygen Determination Method (Part 1). *The JOCS Standard Methods for the Analysis of Fats, Oils and Related Materials 2.3.7.1-2013*
- *Yuki Gosei Kagaku (Synthetic Organic Chemistry),* 1985, vol. 23 (7), 612-619
- Oxirane Oxygen Determination Method (Part 1). *Oxirane oxygen: measured in accordance with The JOCS Standard Methods for the Analysis of Fats, Oils and Related Materials 2.3.7.1-2013*